(19) Europäisches Patentamt European Patent Office Office européen des brevets

(11) EP 2 218 463 A1

(12) EUROPEAN PATENT APPLICATION
published in accordance with Art. 153(4) EPC

(43) Date of publication:
**18.08.2010 Bulletin 2010/33**

(21) Application number: **08843899.9**

(22) Date of filing: **28.10.2008**

(51) Int Cl.:
***A61K 51/00*** *(2006.01)* ***C07D 471/04*** *(2006.01)*

(86) International application number:
**PCT/JP2008/069502**

(87) International publication number:
**WO 2009/057576 (07.05.2009 Gazette 2009/19)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.10.2007 JP 2007282362**

(71) Applicant: **Nihon Medi-Physics Co., Ltd.**
**Tokyo 136-0075 (JP)**

(72) Inventors:
- **TANIFUJI, Shigeyuki**
  **Sodegaura-shi**
  **Chiba 299-0266 (JP)**
- **NAKAMURA, Daisaku**
  **Sodegaura-shi**
  **Chiba 299-0266 (JP)**
- **TAKASAKI, Shinya**
  **Sodegaura-shi**
  **Chiba 299-0266 (JP)**

(74) Representative: **Wilhelms · Kilian & Partner**
**Patentanwälte**
**Eduard-Schmid-Straße 2**
**81541 München (DE)**

(54) **USE OF NOVEL COMPOUND HAVING AFFINITY FOR AMYLOID, AND PROCESS FOR PRODUCTION OF THE SAME**

(57) The invention provides a reagent for detecting amyloid in a biological tissue which can be detect amyloid *in vitro* and *in vivo* with high sensitivity using a compound which has affinity with amyloid and is suppressed in toxicity such as mutagenicity. The reagent for detecting amyloid deposited in a biological tissue comprises a compound represented by the following formula (1) or a salt thereof:

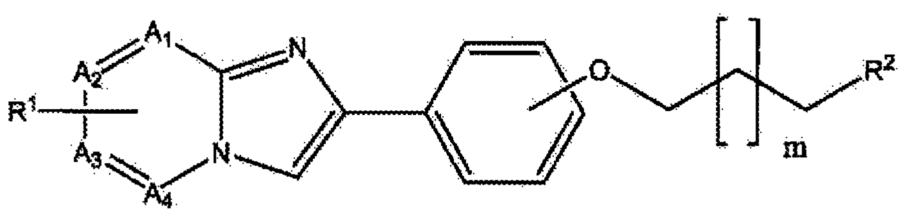

(1)

wherein $A^1$, $A^2$, $A^3$ and $A^4$ independently represents a carbon or nitrogen; $R^1$ is a halogen substituent; $R^2$ is a halogen substituent; and m is an integer or 0 to 2, provided that at least one of $R^1$ and $R^2$ is a radioactive halogen substituent, at least one of $A^1$, $A^2$, $A^3$ and $A^4$ represents a carbon, and $R^1$ binds to a carbon represented by $A^1$, $A^2$, $A^3$ and $A^4$.

EP 2 218 463 A1

## Description

### TECHNICAL FIELD

The present invention relates to use and process for production of a novel compound having affinity for amyloid, and especially relates to a reagent for detecting amyloid in biological tissues, which is useful for detection of amyloid at lesion sites in diagnosis of systemic amyloidosis diseases and other diseases with amyloid accumulation.

### BACKGROUND ART

Diseases with the onset of deposition of a fibrous protein called amyloid in various organs or tissues in bodies are generally referred to as amyloidosis. A feature common to amyloidosis is that the fibrous protein called amyloid which is enriched with the β-sheet structure is deposited at various organs systemically or at sites topically so that functional abnormalities are triggered in the organs or tissues. Amyloid generally refers to protein aggregates formed by aggregation of various amyloid precursor proteins such as amyloidβ, mutant transthyretin and β2-microglpbulin in a living body. The amyloid has a characteristic structure enriched with β-sheet even if it is formed of any of the amyloid precursor proteins. Thus, compounds such as congo-red and thioflavin T capable of binding to β-sheet are **characteristic in that** they have affinity with amyloid.

Amyloidosis is classified into a systemic amyloidosis and a localized amyloidosis depending amyloid deposition patterns.

The systemic amyloidosis is a disease in which amyloid deposition occurs at various parts of the whole body. Examples of the systemic amyloidosis include familial amyloidosis in which amyloid produced in the lever is deposited in organs throughout the whole body so as to cause disorder, senile TTR amyloidosis in which amyloid is deposited in heart and a large joint such as hand joint, dialysis amyloidosis occurring at sites such as bones and joints of long-term dialysis patients, reactive AA amyloidosis (secondary amyloidosis) which is caused by deposition of amyloid derived from serum amyloid A which is an acute phase protein produced following a chronic inflammatory disease such as chronic rheumatism, and immunocytic amyloidosis in which amyloid derived from immunoglobulin is deposited in various organs throughout the whole body.

The localized amyloidosis is a disease in which amyloid deposition occurs only at some organs. Examples of the localised amyloidosis include brain amyloidosis such as Alzheimer's disease in which amyloid is deposited in brain, cerebrovascular amyloidosis and Creutzfeldt Jakob disease, endocrine amyloidosis in which amyloid is deposited in pancreatic inslet accompanied by type II diabetes and insulinoma or deposited in heart atrium, cutaneous amyloidosis in which amyloid is deposited in skin, and localized nodular amyloidosis in which nodulartuberous amyloid is deposited in skin and lungs.

Diagnosis of amyloidosis is, in case of the systemic amyloidosis, at first conducted by collecting a tissue from a region where biopsy is available such as skin, kidney and gastrointestinal tract, and staining it with Congo-red or thioflavin T. Congo-red is a fluorescent compound high in affinity to β-sheet structure of amyloid, and since Congo-red shows doubly refraction under polarization microscope due to its orientation, it can selectively stain amyloid deposition in tissues. Similarly, Thioflavin is also a fluorescent compound having affinity with amyloid, and used similarly to Congo-red. After the tissues staining is found to be positive, definite diagnosis is conducted by means of immunostaining with an antibody or the like in combination therewith. However, positive diagnosis is often difficult by staining with Congo-red and Thioflavin T even under polarization microscopy.

On the other hand, imaging diagnosis of the systemic amyloidosis has been considered with recent wide spread of image diagnosis device such as PET, SPECT and MRI.

However, when Congo-red and Thioflavin T are labeled and used as probes for imaging diagnosis, they are problematic in that binding specificity to amyloid is inferior so that good detection sensitivity cannot be obtained.

Further, since Congo-red has carcinogenicity, it cannot be sued for diagnosis uses of human body.

Thus, bis-(3-hydroxycarbonyl-4-hydroxy)styrylbenzene (BSB) as a congo-red derivative and derivatives thereof have been proposed as a fluorescent reagent for detecting amyloid which is high in affinity and detection sensitivity for amyloid and can be used for *in vivo* detection (non-Patent Document 14, Patent Document 7). It has been reported that BSB is high in affinity for amyloid caused by brain amyloidosis and systemic amyloidosis, and has no no benzizine structure in its structure, and thus it has little carcinogenic problem and can be radioactive-labeled for use as a probe for imaging diagnosis.

on the other hand, for Alzheimer' s disease (hereinafter, referred to as AD) which is a typical brain amyloidosis, attempts have already been made to detect AD *in vivo* using a compound having high affinity with amyloid as a marker since it is impossible to collect a biopsy.

Many of such probes for imaging diagnoses of cerebral amyloid are hydrophobic low-molecular weight compounds that are high in affinity with amyloid and high in cerebral transferability and are labeled with various radioactive species such as $^{11}C$, $^{18}F$ and $^{123}I$. For example, reports tell $^{11}C$ or radioactive halogen labeled forms of compounds including various

thioflavin derivatives such as 6-iodo-2-[4'-(N,N-dimethylamino) phenyl]benzothiazole (hereinafter referred to as TZDM) and 6-hydroxy-2-[4'-(N-methylamino)phenyl]benzothiazole (hereinafter referred to as 6-OH-BTA-1) (Patent Document 1, Non-Patent Document 3); stilbene compounds such as (E)-4-methylamino-4'-hydroxystilbene (hereinafter referred to as SB-13) and (E)-4-dimethylamino-4'-iodostilbene (hereinafter referred to as m-I-SB) (Patent Document 2, Non-Patent Document 4, Non-Patent Document 5); benzoxazole derivatives such as 6-iodo-2-[4'-(N,N-dirnethylamino)phenyl] benzoxazole (hereinafter referred to as IBOX) and 6-[2-(fluoro)ethoxyl]-2-[2-(2-dimethylaminothiazol-5-yl)ethenyl]benzoxazole (Non-Patent Document 6, Non-Patent Document 7), DDNP derivatives such as 2-(1-{6-[(2-fluoroethyl) (methyl) amino]-2-naphthyl}ethylidene)malononitrile (hereinafter referred to as FDDNP) (Patent Document 4, Non-Patent Document 8); and imidazopyridine derivatives such as 6-iodo-2-[4'-(N,N-dimethylamino)phenyl]imidazo[1,2-a]pyridine thereinafter referred to as IMPY) (Patent Document 3,
Non-Patent Document 9). Further, some of these probes for imaging diagnosis have been studied on human imaging and have been reported to show a significant radioactivity accumulation in AD patient's brain compared with normal persons (Non-Patent Document 10, Non-Patent Document 11, Non-Patent Document 12, and Non-Patent Document 13).
International Publication No. WO2007/002540 pamphlet discloses a series of compounds with a group having affinity with amyloid, to which a radioisotope labeling site is attached via ethylene glycol or polyethylene glycol (Patent Document 5).
International Publication No. WO2007/063946 pamphlet discloses a series of compounds to which a five-membered aromatic heterocyclic group is attached in order to prevent them from being metabolized in brain (Patent Document 6).
[Patent Document 1] JP-T-2004-506723
[Patent Document 2] JP-T-2005-604055
[patent Document 3] JP-T-2005-512945
[Patent Document 4] JP-T-2002-523383
[Patent Document 5] International Publication No. WO2007/002540 pamphlet
[Patent Document 6] International Publication No. WO2007/063946 pamphlet
[Patent Document 7] International Publication No. WO2005/016888 pamphlet
[Non-Patent Document 1] J. A. Hardy & G. A. Higgins, "Alzheimer's Disease: The Amyloid Cascade Hypothesis.", Science, 1992, 256, p.184-185
[Non-Patent Document 2] G. McKhahn et al., "Clinical diagnosis of Alzheimer's disease: Report of the NINCDS-ADRDA Work Group under the auspices of Department of Health and Human Services Task Force on Alzheimer's Disease, Neurology, 1984, 34, p.939-944
[Non-Patent Document 3] Z.-P. Zhuang et al., "Radioiodinated Styrylbenzenes and Thioflavins as Probes for Amyloid Aggregates,", J. Med. Chem., 2001, 44, p.1905-1514
[Non-patent Document. 4] Masahiro Ono et al., "11C-labeled stilbene derivatives as Aβ-aggregate-specific PET imaging agents for Alzheimer's disease.", Nuclear Medicine and Biology, 2003, 30, p.565-571
[Non-Patent Document 5] H. F. Kung et al., "Novel Stilbenes as Probes for amyloid plaques.", J. American Chemical Society, 2001, 123, p.12740-12741
[Non-Patent Document 6] Zhi--Ping Zhuang et al., "IBOX (2-(4'-dimethylaminophenyl)-6-iodobensoxazole): a ligand for imaging amyloid plaques in the brain.", Nuclear Medicine and Biology, 2001, 28, p.887-894
[Non-Patent Document 7] Furumoto Y et al., "[11C]BF-227: A New 11C-Labeied 2-Ethenylbenzoxazole Derivative for Amyloid-β Plaques Iniaging.", European Journal of Nuclear Medicine and Molecular Imaging, 2005, 32, Sup.1, P759
[Non-Patent Document 8] Eric D. Agdeppa et al., "2-Dialkylamimp-6-Acylamlononitrile Substituted Naphthalenes (DDNP Analogs): Novel Diagnostic and Therapeutic Tools in Alzheimer's Disease.", Molecular Imaging and Biology, 2003, 5, p.404-417
[Non-Patent Document 9] Zhi-Ping Zhuang et al., "Structure-Activity Relationship of Imidazo[1,2-a]pyridines as Ligands for Detecting β-Amyloid Plaques in the Brain.", J. Med. Chem, 2003, 46, p.237-243 [Non-patent Document 10] W. E. Klunk et al., "Imaging brain amyloid in Alzheimer's disease with Pittsburgh Compound-B.", Ann. Neurol., 2004, 55, p.306-319 [Non-Patent Document 11] Nicolaas P. L. G. Verhoeff et al., "In-Vivo Imaging of Alzheimer Disease β-Amyloid with [11C]SB-13 PET.", American Journal of Geriatric Psychiatry, 2004, 12, p.584-595
[Non-Patent Document 12] Hiroyuki Arai et al., "[11C]-BF-227 AND PET to Visualize Amyloid in Alzheimer's Disease Patients", Alzheimer's & Dementia: The Journal of the Alzheimer's Association, 2006, 2, Sup. 1, S312
[Non-Patent Document 13] Christopher M. Clark et al., "Imaging Amyloid with I123 IMPY SPECT", Alzheimer's & Dementia: The Journal of the Alzheimer's Association, 2006, 2', Sup. 1, S342
[Non-Patent Document 14] D. M. Skovronsky et al., Proc. Natl. Acad. Sci., 2000, 97, 7609

DISCLOSURE OF THE INVENTION

PROBLEMS TO BE SOLVED BY THE INVENTION

As described above, various compounds are disclosed as probes for imaging diagnosis for amyloid, and researched for clinical application.
Experiments in normal mice show that [$^{125}$I]-labeled TZDM, TBOX and m-I-SB are all transferred into brain 2 minutes after administration. However, these compounds are insufficient in clearance from normal tissues, and tend to accumulate gradually in brain as time passes after administration (JP-T-2005-512945; Zhi-Ping Zhuang et al., Nuclear Medicine, and Biology, 2001, 28, p.887-894; H. F. King eft al., J. Am. Chem. Soc., 2001, 123, p.12740-13741). When the clearance from normal tissues is insufficient, a problem arises in that sufficient contrast cannot be obtained at amyloid accumulation sites. [$^{11}$C]-labeled SB-13 shows a clearance from normal tissues in experiments in rats, however, it cannot be said that the clearance is sufficiently fast (Masahiro Ono et al., Nuclear Medicine and Biology, 2003, 30, p.565-571).
Meanwhile, it is revealed that compounds having an imidazopyridine skeleton such as IMPY have a property of transferring to brain and accumulating at amyloid after administration, and also have an excellent property of rapid clearance from normal tissue unlike the above-described compounds, as a result of experiments using [$^{123}$I]-labeled compounds. However, IMPY is a compound positive in reverse mutation test. In order to use this compound as a probe for imaging diagnosis, sufficient care must be taken about dosage and administration manner (International Publication No. WO03/106439 pamphlet).
FDDNP is also reported to be positive in reverse mutation test (International Publication No. WO03/106439 pamphlet).
A preferable probe targeting amyloid for imaging diagnosis would be a compound that is excellent in affinity with amyloid and sufficiently rapid in clearance from normal tissues like IMPY but is suppressed in toxicity such as mutagenicity. However, no compound with such properties has been disclosed.
Furthermore, in accordance with results of our studies, it has been confirmed that IMPY accumulates unspecifically on white matter or other sites where amyloid is not deposited. As an AD diagnostic agent, a compound must be used which is suppressed in unspecific accumulation on sites other than amyloid deposition, but such as compound has not been disclosed.
The present invention has been made under such circumstances where various compounds as probes targeting amyloid have been disclosed but there has been no compound which is confirmed to have a clinically tolerable property, and aims at making it possible to detect amyloid *in vivo* or in vitro with high sensitivity by providing a novel compound having affinity with amyloid.

MEANS FOR SOLVING THE PROBLEM

The present inventors have found that a specific novel compound with an imidazopyridine-phenyl skeleton or a skeleton similar thereto whose phenyl group has a carbon to which an oxygen atom is attached has affinity with amyloid and is low in toxicity such as mutagenicity, and amyloid can be detected *in vivo* or *in vitro* with high sensitivity by use of a series of the compound as a probe. Thus, the present invention has been completed.
That is, according to one aspect of the present invention, a reagent for detecting amyloid deposited on a biological tissue is provided, which comprises a compound represented by the following formula (1):
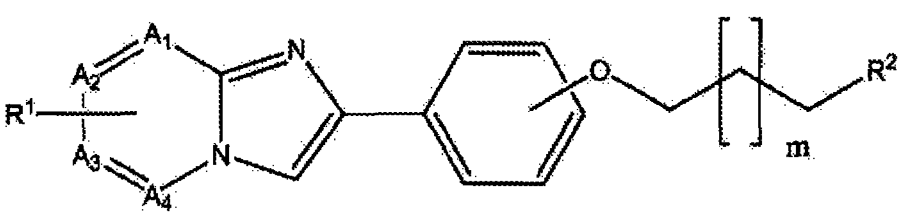

(1)

or a salt thereof.
The biological tissue can be various tissues at which amyloid is known to deposit in amyloidosis.
Typical examples of such biological tissues include brain, heart, lung, pancreas, bone and joint, and as the most typical biological tissue, mention may be made of brain. The typical amyloidosis in case of brain include Alzheimer's disease and dementia with Lewy bodies.
In the formula (1), $R^1$ and $R^2$ are halogen substituents, and at least either of them is a radioactive halogen

substituent. Various nuclides can be used as the halogen, and fluorine, bromine or iodine can be preferably used. As the radioactive halogen, can be used various elements, preferably a halogen selected from $^{18}F$, $^{75}Br$, $^{76}Br$, $^{123}I$, $^{124}I$, $^{125}I$ or $^{131}I$, and more preferably a halogen selected from $^{18}F$, $^{123}I$ or $^{125}I$.

$A_1$, $A_2$, $A_3$ and $A_4$ independently represent a carbon or nitrogen, and it is necessary that at least one of these represents a carbon. Preferably, 3 or more of $A_1$, $A_2$, $A_3$ and $A_4$ represent carbons, and more preferably, all of them represent carbons. In the formula (1), $R^1$ binds to a carbon represented by $A_1$, $A_2$, $A_3$ or $A_4$.
In addition, m is an integer of 0 to 2. A binding site for $R^1$ is preferably a carbon represented by $A_3$, that is, a carbon at 6-position.

The compound above in the formula (1) is a novel compound, and according to another aspect of the present invention, a process for production of a radioactive halogen-labeled organic compound is provided, which comprises a step of preparing a reaction solution containing, together with a radioactive halogen ion, a compound represented by the following formula (2):

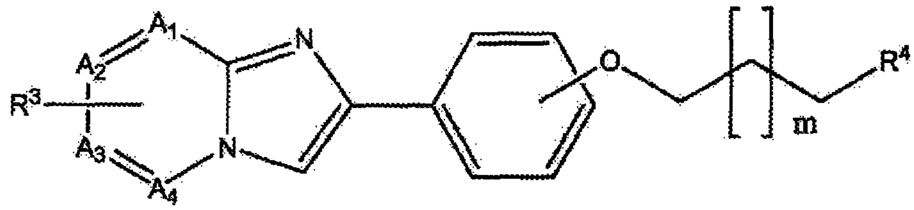

(2)

wherein, $A_1$, $A_2$, $A_3$ and $A_4$ independently represent a carbon or nitrogen, $R^3$ is a group selected from the group consisting of a npn-radioactive halogen substituent, nitro substituent, trialkylammonium substituent having from 1 to 4 carbon atoms, trialkylstannyl substituent having from 1 to 4 carbon atoms and triphenylstannyl substituent, $R^4$ is a group selected from the group consisting of a non-radicactive halogen substituent, methanesulfonyloxy substituent, trifluororoethanesulfonyloxy substituent and aromatic sulfonyloxy substituent, and m is an integer of 0 to 2, provided that at least one of $A_1$, $A_2$, $A_3$ and $A_4$ represents a carbon, and $R^3$ binds to a carbon represented by $A_1$, $A_2$, $A_3$ or $A_4$, or a salt thereof, and step of giving a reaction condition to the reaction solution to synthesize a compound represented by the following formula (1):

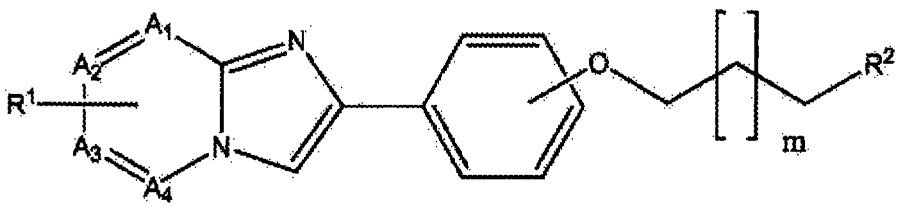

(1)

wherein $A_1$, $A_2$, $A_3$ and $A_4$ independently represent a carbon or nitrogen, $R^1$ is halogen substituent, $R^2$ is halogen substituent, and m is an integer of 0 to 2, provided that at least one of $R^1$ and $R^2$ is a radioactive halogen substituent, at least one of $A_1$ $A_2$, $A_3$, and $A_4$ represents a carbon, and $R^1$ binds to a carbon represented by $A_1$, $A_2$, $A_3$ or $A_4$, or a salt thereof.

The formula (2), $R^3$ is a group selected from the group consisting of a non-radioactive halogen substituent, nitro substituent, trialkylammonium substituent having alkyl chains with 1 to 4 carbon atoms, trialkylstannyl substituent having alkyl chains with 1 to 4 carbon atoms and triphehylstannyl substituent. As the trialkylstannyl substituent, various substituents can be used, and trimethylstannyl substituent and tributylstannyl substituent are preferably used.

$R^4$ is a group selected from the group consisting of a non-radioactive halogen substituent, methanesulfonyloxy substituent, trifluoromethanesulfonyloxy substituent and aromatic sulfonyloxy substituent. As the aromatic sulfonyloxy substituent, toluenesulfonic acid, nitrobenzenesulfonic acid and benzenesulfonic acid can be preferably used.

As a non-radioactive halogen substituent of $R^3$ and $R^4$, various halogens can be used, but preferably a halogen capable of being a target of nucleophilic substitution reactions using a radioactive fluorine or a halogen capable of being a target of isotope exchange reactions with a radioactive iodine can be used, and more preferably chlorine, iodine or bromine can be used. At least one of $R^3$ and $R^4$ is preferably the non-radioactive halogen substituent.

$A_1$, $A_2$, $A_3$ and $A_4$ independently represent a carbon or nitrogen, and it is necessary that at least one of these represents a carbon. Preferably, 3 or mare of $A_1$, $A_2$, $A_3$ and $A_4$ represent carbons, and more preferably, all of them represent carbons. In the formula (2), $R^3$ binds to a carbon represented by $A_1$, $A_2$, $A_3$ or $A_4$.
In addition, m is an integer of 0 to 2.

Further, according to the present invention, there is provided a compound with an imidazopyridine-phenyl skeleton in which a carbon atom of the phenyl group is bonded via an oxygen atom to an alkyl chain which is substituted at the terminal thereof, but a binding site of the oxygen is preferably a carbon at 4'-position of the phenyl group. Also, a binding site for $R^3$ is preferably a carbon represented by $A_3$, that is, a carbon at 6-position.

The step of preparing a reaction solution comprising a precursor compound shown above in the formula (2) or a salt thereof and a radioactive halogen ion can be conducted, for example, by dissolving the precursor compound or a salt thereof in an inert organic solvent, and adding thereto a radioactive halogen, ion-containing solution which has been obtained with a known method.
As the inert organic solvent, various solvents which do not have reactivity with the precursor compound or a salt thereof and the radioactive halogen ion can be used, for example, when a radioactive halogen ion to be used is a radioactive iodine, methanol can preferably be used, and when a radioactive halogen ion to be used is a radioactive fluorine, acetonitrile can preferably be used.
The reaction condition to be given for the reaction solution in the step of synthesizing a compound represented above in the formula (1) or a salt thereof is not particularly limited as long as it is a condition in which a reaction of substituting $R^3$ or $R^4$ of a compound of the formula (2) with the radioactive halogen ion added in the reaction solution is allowed to proceed, and a known reaction condition that fits kinds of the radioactive halogen ion can be used.

In the process for producing the radioactive halogen-labeled organic compound of the present invention, as the radioactive halogen ion, for example, $^{18}F$, $^{75}Br$, $^{76}Br$, $^{123}I$, $^{124}I$, $^{125}I$ or $^{131}I$ can be used.
When a compound of the formula (1) in which a halogen substituent represented by $R^1$ is it $^{124}I$, $^{125}I$ or $^{131}I$ is produced, $^{123}I$ ion, $^{124}I$ ion, $^{125}I$ ion or $^{131}I$ ion is respectively used as a radioactive halogen ion, and as a compound of the formula (2), preferably used is a compound in which $R^3$ is a non-radioactive iodine, trialkylstannyl substituent having alkyl groups with from 1 to 4 carbon atoms or triphenylstannyl substituent (in this case, $R^2$ and $R^4$ are non-radioactive halogen substituents).
When a compound of the formula (1) in which a halogen substituent represented by $R^1$ is $F^{13}$ is produced, $F^{18}$ ion is used as a radioactive halogen ion and as a compound of the formula (2), preferably used is a compound in which $R^3$ is a nitro substituent or trialkylammonium substituent having alkyl groups with from 1 to 4 carbon atoms (in this case, $R^2$ and $R^4$ are non-radioactive halogen substituents).
When a compound of the formula (1) in which a halogen substituent represented by $R^1$ is $^{75}Br$ or $^{76}Br$ is produced, $^{75}Br$ ion or $^{76}Br$ ion is respectively used as a radioactive halogen ion, and as a compound of the formula (2), preferably used is a compound in which $R^3$ is a non-radioactive bromine (in this case, $R^2$ and $R^4$ are non-radioactive halogen substituents).
When a compound of the formula (1) in Which a halogen substituent represented by $R^2$ is $F^{18}$ is produced, $F^{18}$ ion is used as a radioactive halogen ion and as a compound of the formula (2), preferably used is a compound in which $R^4$ is methanesulfonyloxy substituent, trifluoromethanesulfonyloxy substituent or aromatic sulfonyloxy substituent (in this case, $R^1$ and $R^3$ are non-radioactive halogen substituents).
When a compound of the formula (1) in which a halogen substituent represented by $R^2$ is $^{123}I$, $^{124}I$, $^{125}I$ or $^{131}I$ is produced, $^{123}I$ ion, $^{123}I$ ion, $^{125}I$ ion or $^{131}I$ ion is respectively used as the radioactive halogen ion, and as a compound of the formula (2), preferably used is a compound in which $R^4$ is a non-radioactive iodine (in this case, $R^1$ and $R^3$ are non-radioactive halogen substituents).
When a compound of the formula (1) in which a halogen substituent represented by $R^2$ is $^{75}Br$ or $^{76}Br$ is produced, $^{75}Br$ ion or $^{76}Br$ ion is respectively used as a radioactive halogen ion, and as a compound of the formula (2), preferably used is a compound in which $R^4$ is a non-radioactive bromine (in this case, $R^1$ and $R^3$ are non-radioactive halogen substituents).

According to still another aspect of the present invention, a precursor compound for synthesizing a radioactive halogen-labeled organic: compound is provided, which comprises a compound represented by the following formula (2):

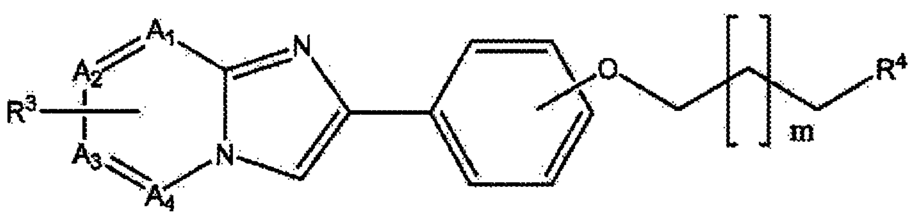

(2)

wherein $A_1$, $A_2$, $A_3$ and $A_4$ independently represent a carbon or nitrogen, $R^3$ is a group selected from the group consisting of a non-radioactive halogen substituent, nitro substituent trialkylammonium substituent having alkyl chains with from 1 to 4 carbon atoms, trialkylstannyl substituent having alkyl chains with from 1 to 4 carbon atoms or triphenylstannyl substituent, $R^4$ is a group selected from the group consisting of a non-radioactive halogen substituent, methanesulfonyloxy substituent, trifluoromethanesulfonyloxy substituent and aromatic sulfonyloxy substituent, and m is an integer of 0 to 2, provided that at least one of $A_1$, $A_2$, $A_3$ and $A_4$ represents a carbon, and $R^3$ binds to a carbon represented by $A_1$, $A_2$, $A_3$ and $A_4$, or a salt thereof.

In the formula (2), the preferable embodiments of $A_1$, $A_2$, $A_3$ and $A_4$, and $R^3$ and $R^4$ are the same as described above concerning the production process.

## EFFECT OF THE INVENTION

In accordance with the present invention, a reagent for detecting amyloid, which has affinity with amyloid and is suppressed in toxicity such as mutagenicity and thus can be used for *in vitro* and *in vivo* detection of amyloid related to a wide range of amyloidosis, can be obtained as well as process for production thereof and a production intermediate thereof.

## BEST MODE FOR CARRYING OUT THE INVENTION

(A method for synthesis of a precursor compound for a radioactive halogen-labeled compound)

Hereinafter, a method for synthesis of a precursor compound for preparing a radioactive halogen-labeled organic compound according to an aspect of the present invention will be described, taking the case of 6-tributylstannyl-2-[4'-(3''-fluoropropoxy)phenyl]imidazo[1,2-a]pyridine.

First, 4'-hydroxyacetophenone is allowed to react with cupric bromide to prepare 2-bromo-4'-hydroxyacetophenone (Fig. 1-1, Step 1). In this instance, the reaction can be conducted in accordance with ordinary methods, for example, the method described in a literature, King, L. Carroll and Ostrum, G. Kenneth, Journal of Organic Chemistry, 1964, 29(12), p.3459-3461.

Then, 2-bromo-4'-hydroxyacetophenone as prepared above is allowed to react with 2-amino-5-bromopyridine to prepare 6-bromo-2-(4'-hydroxyphenyl)imidazo[1,2-a]pyridine (Fig. 1-1. Step 2). This step can be done according to the following procedure.

First, 2-bromo-4'-hydroxyacetophenone and 2-amino-5-bromopyridine are dissolved in an inactive solvent such as acetonitrile, and are allowed to react with each other at a reflux temperature for 2 to 6 hours to produce 6-bromo-2-(4'-hydroxyphenyl)imidazo[1,2-a]pyridine hydrobromide salt as white precipitates. The solvent used in this instance may be acetonitrile or anther solvent that is usually employed in a similar reaction, for example, methanol and acetone. The reaction temperature may be a temperature allowing refluxing, for example, 90˚C when the solvent is acetonitrile. The amount of the solvent to be used may be an amount sufficient to effect the reaction, however, it should be noted that if the solvent is too much, it will become difficult to obtain precipitates of reaction products. For example, when 2-bromo-4'-hydroxyacetophenone in an amount corresponding to 10 mmol is used for the reaction, the amount of a solvent to be used can be about 40 to 50 mn.

Next, the reaction solution is filtered to recover the precipitates. The white precipitates are suspended in a mixed solution of methanol/water (1:1). Then, an aqueous saturated solutions of sodium hydrogencarbonate is added thereto in a very excessive amount relative to the suspended precipitates to release 6-bromo-2-(4'-hydroxyphenyl) imidazo[1,2-a]pyridine as precipitates.
The newly generated precipitates are filtered to recover 6-bromo-2-(4'-hydroxyphenyl)imidazo[1.2-a]pyridine as the target compound in this step (Fig. 1-1, Step 2). The amount of the mixed solution of water/methanol is not specifically limited as long as it is sufficient to effect the reaction. However, it should be noted that if the amount of the mixed solution is too much, precipitation of products will be hindered. For example, when 2-'bromo-4'-hydroxyacetophenone in an amount corresponding to 10 mmol is used, the mixed solution of water/methanol may be used in an amount of about 40 to 100 mL. The amount of sodium hydrogencarbonate is not specifically limited as long as it is very excessive relative to the above-described precipitates as reaction substrates. For example, when the reaction is effected under the above-described conditions, the amount of an aqueous saturated solution of sodium hydrogencarbonate to be added to the reaction solution can be about 25mL.

Then, the 6-bromo-2-(4'-hydroxyphenyl)imidazo[1,2-a] pyridine prepared above is sufficiently dried, dissolved in N,N-dimethylformanide, and potassium carbonate and 3-bromo-1-fluoropropane were added thereto. The reaction solution is stirred at room temperature for overnight to obtain 6-bromo-2-[4'-(3''-fluoropropoxy)phenyl]imidazo[1,2-a] pyridine (Fig. 1-1, Step 3). The amount of potassium carbonate to be used may be an amount that can neutralise hydrobromic acid generated from 3-bromo-1-fluoropropane during the reaction, and is typically about double the other

reactant 3-bromo-1-fluoropropane in molar ratio. The 3-bromo-1-fluoropropane can be used in an excessive amount relative to the reaction substrate, and is typically about 1.5 times the reaction substrate 6-bromo-2-(4'-hydroxyphenyl) imidazo[1,2-a]pyridine in molar ratio.

The obtained 6-bromo-2-[4'-(3"-fluoropropoxy)phenyl]imdazo[1,2-a]pyridine was dissolved in dioxane, and after triethylamine is added, bis(tributyltin) and a catalytic amount of tetrakis-triphenylphosphine palladium are added. This reaction mixture is heated at about 90°C and reacted for about 24 hours, and then a solvent is distilled off and a chromatographic purification is performed to obtain 6'-tributylstannyl-2-[4'-(3"-fluoropropoxy)pnenyl]imdazo[1,2-a]pyridine as the target compound (Fig. 1-1, Step 4). The amount of bis(tributyltin) to be used in this instance may be an amount satisfying a condition where it is excessive relative to the reaction substrate, specifically, it is about 1.5 times in molar ratio relative to the reaction substrate 6-bromo-2-[4'-(3"-fluoropropoxy)phenyl]imidazo[1,2-a]pyridine,

When a compound, with a substituent at the 6-postion being a trialkylstannyl substituent other than tributylstannyl substituent is obtained, various bis(trialkyltin)s that fist purposes can be used instead of bis(tributyltin) in Step 4. For example, when a compound having a trimethylstannyl substituent as a substituent at the 6-position is synthesized, a reaction similar to the above can be performed in Step 4 using bis(trimethyltin).

A compound with an imidazopyridine ring in which the binding site for the functional group is a carbon atom other than the carbon at 6-pqsition can be obtained by using a compound with a pyridine ring to which bromine is bonded at a different site instead of 2-amino-5-bromopyridine used in Step 2. For example, when a binding site for the functional group is the carbon at 8-position in the imidazopyridine ring, 2-amino-3-bromopyridine may be used instead of 2-amino-5-bromopyridine in Step 2.

Further, a precursor compound with a radioactive halogen labeled site being an alkoxyphenyl substituent attached to a carbon atom at 2-position of the imidazo pyridine ring can be obtained by using 3-bromo-1-propanol instead of 3-bromo-1-fluoropropane, and reacting a resulting compound with p-toluenesulfonylchloride or the like. For example, 6-bromo-2-[4'-(3"-p-toluenesulfonyloxypropoxy)phenyl]imidazo[1,2-a]pyridine can be synthesized by the following procedure.

First, 6-bromo-2-(4'-hydroxyphenyl)imidazo[1,2-a]pyridine prepared above is dissolved in N,N-dimethylformamide, and the solution is supplemented with potassium carbonate and 3-bromo-1-propanol and stirred at room temperature for overnight to obtain 6-bromo2- [4'-(3"-hydroxypropoxy)phenyl]imidazo[1,2-a]pytidine. This is dissolved in pyridine, supplemented with p-toluenesulfonylchloride under an ice bath and then reacted at room temperature to obtain 6-bromo-2-[4'-(3"-p-toluenesulfonyloxypropoxy)phenyl]imidazo[1,2-a]pyridine as a target compound. The amount of the p-toluenesulfonylchloride to be used in this instance may be excessive relative to the reaction substrate, and is typically about double the reaction substrate 6-bromo-2-[4'-(3"-hydrxypropoxy)phenyl]imidazo[1,2-a]pyridine in molar ratio.

A compound in which the alkoxy substituent bound to the phenyl group that is bound to the 2-positipn of the imidazopyridine ring is bound to another position than the 4'-position, for example, 6-tributylstannyl-2-[3'-(3"-fluoropropoxy)phenyl]imidazo[1,2-a]pyridine which has a fluoropropoxy group at 3'-position, can be synthesized in accordance with the same reaction as above except using 3'-hydroxyacetophenone as a reactant instead of 4'-hydroxyacetophenone in step 1.

(A method for synthesis of a radioactive halogen-labeled organic compound)

Hereinafter, a method for production of a radioactive halogen-labeled organic compound according to another aspect of the present invention will be described, taking the case of 2-[4'-(3"-fluoropropoxy)phenyl]-6-[$^{123}$I]iodoirnidazo [1,2-a]pyridine.

For then production of 2-[4'-(3"-fluorropropoxy)phenyl]-6-[$^{123}$I]iodoimidazo[1,2-a]pyridine,
a [$^{123}$I]sodium iodide solution to be served for labeling is first obtained. A [$^{123}$I]radioactive iodine can be obtained by, for example, a known method in which a xenon gas is used as a target and exposed to proton bombardment, This [$^{123}$I] radioactive iodine is made into [$^{123}$I]sodium iodide solution by using known methods, and used for the labeling.

Then, the labeling precursor 6-tributylstannyl-2-[4'-(3"-fluoropropoxy)phenyl]imidazo[1.2-a]pyridine is dissolved in an inert organic solvent, and a solution of the [$^{123}$I]iodo bulk dissolved in water, an acid and an oxidizing agent are added thereto and allowed to react to obtain 2-[4'-(3"-fluoropropoxy)phenyl]-6-[$^{123}$I] iodoimidazo[1,2-a]pyridine as a target compound. As the inert organic solvent dissolving the precursor compound, various solvents having no reactivity with the labeling precursor and [$^{113}$I]iodo bulk can be used, and preferably methanol can be used.

As the acid, may be used various ones, and preferably hydrochloric acid.
The oxidizing agent is not particularly limited as long as it can effect the oxidation of iodine in the reaction solution, and is preferably hydrogen peroxide or peracetic acid. The amount of the oxidizing agent to be added may be an amount sufficient to oxidize iodine in the reaction solution.

A compound labeled with a radioactive halogen other than iodine can be synthesized by labeling a labeling precursor that fits a purpose of synthesis with a radioactive halogen that fits the purpose. For example, in order to synthesize 2-[4'-(3"-[$^{16}$F]fluoropropoxy)phenyl]-6-bromoimidazo[1,2-a]pyridine, the labeling precursor 6-bramo-2-[4'-(3"-p-toluenesulfonyloxypropoxy)pheyl]indazo[1.2-a]pyridine can be reacted with [$^{18}$F]fluoride ion the presence of a phase transfer catalyst and potassium carbonate.

(Methods for preparing and using a detection reagent in accordance with the present invention)

Amyloid has many different structures depending upon kinds of precursor protein, but they are common in a point of having β-sheet structure. It has been known that many staining reagents for amyloid such as Thioflavin T and Congo-red target the β-sheet structure, and have the staining ability equally to different kinds of amyloid.
The compound of the formula (1) according to the present invention has affinity with amyloid originated from amyloid β-protein (hereinafter referred to as Aβ) as a precursor compound, and also has an activity of inhibiting the binding to amyloid of Thioflavin T which is known to bind to a wide range of amyloid.
Therefore, the compound of the formula (1) according to the present invention is considered to have affinity with β-sheet structure of amyloid protein like thioflavin T. This suggests that the compound of the formula (1) according to the present invention has the same affinity with various amyloids.
That is, the reagent for detecting amyloid according to the present invention can be used for diagnosing the systematic amyloidosis and localized amyloidosis similarly to Thioflavin T and Congo-red. The systemic amyloidosis includes immunoglobulin amyloidosis, reactive AA amyloidosis, familiar amyloidosis, dialysis amyloidosis and senile amyloidosis. The localized amyloidosis includes brain amyloidosis, endocrine amyloidosis, cutaneous amyloidosis and localized nodular amyloidosis.
The reagent for detecting amyloid according to the present invention can be not only used as a reagent used *in vitro* for biopsy, but also used as a reagent used *in vivo* as a radioactive diagnostic agent since a compound suppressed in toxicity such as mutagenicity is used.
The reagent for detecting amyloid according to the present invention can be prepared as a solution which comprises the radioactive halogen-labeled compound of the above formula (1) blended in water, a physiological saline solution or a Ringer's solution optionally adjusted to an appropriate pH, like other commonly-known radioactive diagnostic agents. In this instance, concentration of the present compound should be adjusted to not more than the concentration at which stability of the present compound is ensured. Dosage of the present compound is not specifically limited as long as it is sufficient to obtain an image of distribution of an administered agent. For example, in case of iodine-123($^{123}$I)-labeled compounds and fluorine-18($^{18}$F)-labeled compounds, about 50 to 600 MBq per adult body of 60 kg weight can be administered intravenously or locally. Distribution of administered agents can be imaged by known methods. For example, iodine-123($^{123}$I)-labeled compounds can be imaged by a SPECT apparatus while fluorine-18($^{18}$F)-labeled compounds can be imaged by a PET apparatus.
By administering the reagent for detecting amyloid according to the present invention to at living body, amyloid which is deposited in biological tissues such as brain, heart, lung, digestive tract, blood vessel, liver, pancreas, kidney, joints and bones can be imaged, and it is useful for imaging amyloid deposition in biological tissues difficult in biopsy collection, for example, brain, heart, lung, pancreas, bone and joint.

EXAMPLE

In the following Examples, the names of the individual compounds used in the experiment are defined as shown in Table 1-1.
Table 1-1

| Compound name | Common name |
|---|---|
| Compound I-1 | 6-bromo-2-[4'-(3"-fluoropropoxy)phenyl]imidazo[1,2-a]pyridine |
| Compound I-2 | 2-[4'-(3"-fluoropropoxy)phenyl]-6-iodoimidazo[1,2-a]pyridine |
| Compound I-3 | 6-bromo-2-[4'-(2"-fluoroethoxy)phenyl]imidazo[1.2-a]pyridine |
| Compound I-4 | 2-[4'-(2"-fluoroethoxy)phenyl]-6-iodoimidazo[1.2-a]pyridine |
| Compound I-5 | 2-[4'-(3"-fluoropropoxy)phenyl]-6-iodoimidazo[1,2-a]pyrimidine |
| Compound I-6 | 2-[4'-(3"fluoropropoxy)phenyl]-6-[$^{125}$I]iodoimidazo[1,2-a]pyridine 4 |
| Compound I-7 | 2-[4'-(3"-fluoropoxy)phenyl]-6-[$^{123}$I]iodoimidazo[1.2-a]pyridine |
| Compound I-8 | 6-bromo-2-[4'-(3"-[$^{18}$F]fluoropropoxy)-phenyl]imidazo[1,2-a]pyridine |

(continued)

| Compound name | Common name |
|---|---|
| Compound I-9 | 2-[4'-(2"-fiuoroethoxy)phenyl]-6-[$^{123}$I]iodoimidazo[1,2-a]pyridine |

Example 1-1: Synthesis of 6-tributylstannyl-2-[4'-(3"-fluoropropaxy)phenyl]imdazo[1,2-a]pyridine

50 mL of ethyl acetate was added, to 28.17 g (corresponding to 126 mmol) of cupric bromide to obtain a suspension, to which a solution of 8.18 g (corresponding to 60.0 mmol) of 4,'-hydrroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated, under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chlofoform/methanol = 20/1), and recrystallized from ethyl acetate/petroleum ether, to obtain 7.25 g (corresponding to 33.7 mmol) of 2-bromo-4'-hydroxyacetophenone (Fig. 1-1, Step 1).

2.15 g (corresponding to 10.0nmol) of 2-bromo-4'-hydroxyacetophenone and 1.74 g (corresponding to 10.0 mmol) of 2-amino-5-bromopyridine were dissolved in 50 mL of acetonitrile. The resulting solution was refluxed in an oil bath at 105°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture was sonicated for 5 minutes using an ultrasonic washing machine. Precipitates were filtered and recovered from the resulting-mixture, sufficiently washed with water, and dried under reduced pressure, to obtain 2.41 g (corresponding to 8.32 mmol) of 6-bromo-2-(4'-hydrroxyphenyl)imidazo[1,2-a]pyridine (Fig. 1-1, Step 2).

290 mg (corresponding to 1.0 mmol) of 6-bromo-2-(4'-hydroxyphenyl)imidazo[1,2-a]pyridine that was sufficiently dried to remove moisture was dissolved in 10 mL of N,N-dimethylformamide, and 413 mg (corresponding to 3.0 mmol) of potassium carbonate was added thereto. The mixture was supplemented with 138 $\mu$L (corresponding to 1.5 mmol) of 1-bromo-3-fluoroproppane, and then was stirred at room temperature for 20.5 hours. After the completion of the reaction, the reaction solution was poured into water and extracted three times with chloroform. The combined chloroform layer was washed with a saturated sodium chloride solution, dried over anhydrous sodium sulfate, filtered and concentrated. The resulting crude product was purified by recycle preparative HPLC (HPLC apparatus: LC-90B (under trade name; manufactured by Japan Analytical Industry Co., Ltd.); column: two JAIGEL 2H (under trade name; Manufactured by Japan Analytical Industry Co., Ltd.) connected together; mobile phase: chloroform), to obtain 302 ing (corresponding to 0.866 mmol) of 6-bromo-2-[4'-(3"-fluoropropoxy)phenyl]imidazo[1,2-a]pyridine (Fig. 1-1, Step 3).

85 mg (corresponding to 0.24 mmol) of 6-bromo-2-[4'-(3"-fluoropropoxy)phenyl]imidazo[1,2-a]pyridine was dissolved in 10 mL of dioxane, and 2 mL of triethylamine was added thereto. Then, 185 $\mu$L (corresponding to 0.36 mmol) of bis (tributyltin) and 20 mg (at a catalytic amount) of tetrakis-triphenylphosphine palladium were added thereto. After the reaction mixture was stirred at 90°C for 24 hours the solvent was distilled off under reduced pressure. The residue was purified by the preparative TLG (elution solvent: hexane/ethyl acetate = 6/4). Further, the resulting crude product was purified by recycle preparative HPLC (HPLC apparatus: LG-908 (under trade name: manufacture by Japan Analytical Industry Co., Lid.); column: two JAIGEL 2H (under trade name; manufactured by Japan Analytical Industry Co., Ltd.) connected together; mobile phase: chloroform), to obtain 42 mg (corresponding to 74.2 $\mu$mol) of 6-tributylstannyl-2-[4' (3"-fluoropropoxy)phenyl]imidazo[1,2-a]pyridine (Fig. 1-1, step 4).

The NMR measurement results of the resulting 6-tributylstannyl-2-[4'-(3"-fluoropropoxy)phenyl]imidazo[1,2-a] pyridine (infernal standard: tetramethylsilane:) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): $\delta$ 8.01-7.93 (m, 1H), 7.91-7.87 (m, 2H), 7.75-7.74 (m, 1H), 7.63-7.58 (m, 1H), 7.20-7,11 (m, 1H), 7.00-6.95 (m,2H), 4.67 (dt, $J_{HF}$ = 47.0 Hz, J = 6.0 Hz, 2H), 4.15 (t, J = 6.0 Hz, 2H), 2.20 (dquint, $J_{HF}$ = 26.1 Hz, J = 6.0 Hz, 2H), 1.64-1.47 (m, 6H), 1.39-1.31 (m, 6H), 1.19-1.04 (m, 6H), 0.91 (t, J = 7.2 Hz, 9H

Example I-2: Synthesis of 2-[4'-(3"-fluoropropoxy)phenyl]-6-[$^{123}$I]iodoimidazo[1,2-a]pyridine

To 53 $\mu$L of a solution of 6-tributylstannyl-2-[4'-(3"-fluoropropoxy)phenyl]imidazo[1,2-a]pyridine in methanol (concentration: 1 mg/mL), 100 $\mu$L of 1 mol/L hydrochloric acid, [$^{125}$I]sodium iodide of 11.1 MBq (20 $\mu$L in volume) and 10 $\mu$L of 10% (w/v) hydrogen peroxide were added. After the mixed solution was left to stand at room temperature for

10 minutes, the solution was subjected to HPLC under the following conditions, to obtain 2-[4'-(3''-fluorpropoxy)phenyl]-6-[$^{125}$I]iodoimidazo[1,2,-a]pyridine fraction.

HPLC conditions:

Column: Phenomenex Luna C18 (trade name; manufactured by Phenomenex Co.; size: 4.6 x 150 mm)
Mobile phase: 0,1 % trifluoroacetic acid in water/0.1 % trifluoroacetic acid in acetonitrile = 80/20 to 0/100 (17 minutes, linear gradient)
Flow rate: 1.0 mL/min.
Detector: Ultraviolet visible absorptiometer (Detection wavelength: 282 nm) and radioactivity counter (manufactured by raptest: type STEFFI)

10 ml of water was added to the fraction. The resulting solution was passed through a reversed phase column (trade name: Sep-Pak (registered trademark) Light C18 Cartridges Manufactured by Waters: the packed amount of the packing agent: 130 mg) so that the column adsorbs and collects 2-[4'-(3''-fluoropropoxy)phenyl]-6-[$^{125}$I] iodoimidazo [1,2,-a]pyridine. The column was rinsed with 1 mL of water, and then 1 mL of ethanol was passed therethrough to elute 2-[4'-(3''-fluoropropoxy)phenyl]-6-[$^{125}$I]iodoimidazo[1,2,-a]pyridine. The amount of radioactivity of the obtained compound was 5.5 MBq (at the end of production). Further, the TLC analysis was conducted under the following conditions, and as a result, the radiochemical purity of the compound was 96.0%.

TLC analysis conditions:

TLC plate: RP-18F254 (trade name; manufactured by Merck & Co., Inc.)
Mobile phase: methanol/water = 20/1
Detector: Bio-imaging Analyzer, BAS-2500 (type: BAS-2500 manufactured by FUJIFILM Corporation)

Example I-3: Synthesis of 2-(4'-(3''-fluoropropoxy)phenyl)-6-[123I]iodoimidazo[1,2,-a]pyridine

To 70 μL of a solution of 6-tributylstannyl-2-[4'-(3''-fluoropropoxy)phenyl]imidazo[1,2-a]pyridine in methanol (concentration: 1 mg/mL), 100 μL of 1 mol/L hydrochloric acid, [$^{123}$I]sodium iodide of 260-330 MBq (30-60 μL in volume), 20 μL of 1 mmol/L sodium iodide solution and 20 μL of 10% (w/v) hydrogen peroxide were added. After the mixed solution was heated at 50°C for 10 minutes, the solution was subjected to HPLC under the same conditions as in Example 1-2, to obtain 2-[4'-(3''-fluoropropoxy)phenyl]-6-[$^{123}$I]iodoimidazo[1,2-a]pyridine as a fraction.

10 ml of water was added to the fraction. The resulting solution was passed through a reversed phase column (trade name: Sep-Pak (registered trademark) Light C18 Cartridges manufactured by Waters: the packed amount of the packing ages: 130 mg) so that the column adsorbs and collects 2-[4'-(3''-fluoropropoxy)phenyl]-6 [$^{123}$I]iodoimidazo[1,2,-a] pyridine. The column was rinsed with 1 mL of water, and then 1 mL of ethanol was passed therethrough to elute 2-[4'-(3''-fluoropropoxy)phenyl]-6 [123I]iodoimidazo[1,2,-a]pyridine. The amount of radioactivity of the obtained compound was 112-153 MBq at the end of synthesis. Further, the TLC analysis was conducted under the same conditions as in Example 1-2, and as a result, the radiochemical purity of the compound was 97.0%.

Example I-4: synthesis of 6-bromo-2-[4'-(3''-p-toluenesulfonyloxypropoxy)phenyl]imidazo[1,2-a]pyridine

50 mL of ethyl acetate was added to 28.17 to (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting: filtrate was concentrated under reduced pressure. The residue was dissolved in ethyl acetate and subjected so decoloring operation with addition of active charcoal. Then, the resulting solution was filtered and concentrated. The resulting crude: product was purified by flash silica gel column chromatography (elution solvent: chloroform/methanol = 20/1), and recrystallized from ethyl acetate/petroleum ether, to obtain 7.25 g (corresponding to 33.7 mmol) of 2-promo-4'-hydroxyacetophenone (Fig. 1-2, step 1).

2.15 g (corresponding to 10.0 mmol) or 2-bromo-4'-hydroxyacetophenone and 1.74 g (corresponding to 10.0 mmol) of 2-amino-5-bromopyridine were dissolved in 50 mL of acetonitrile. The resulting solution was refluxes in an oil bath at 105°C for 6 hours. After the completion of the reaction, the reaction solution wars cooled down to room temperatures and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added: thereto, and the mixture was sonicated for 5 minutes using an ultrasonic washing machine, Precipitates were filtered and recovered from the resulting mixture, sufficiently washed with watery and dried under reduced pressure, to obtain 2.41 g (corresponding to

8.32 mmol) of 6-bromo-2-(4'-hydroxyphenyl)imidazo[1,2-a]pyridine (Fig. 1-2, Step 2).

1.45 g (corresponding to 5.0 mmol) of 6-bromo-2-(4'-hydroxyphenyl)imidazo[1,2-a]pyridine that was sufficiently dried to remove moisture was dissolved on 50 mL of N,N-dimethyrformamide, and 2.07 g (corresponding to 15.0 mol) of potassium carbonate was added thereto. The mixture was supplemented with 680 μL (corresponding to 7.5 mmol) of 3-bromo-1-propanol, and then the solution was stirred at room temperature for 17 hours. After the completion: of the reaction, the reaction solution was poured into water and extracted three times with chloroform. The combined chloroform layer was washed with a saturated sodium chloride solution, dried over anhydrous sodium sulfate, filtered and concentrated. The resulting crude product was recrystallized from methanol to obtain 1.28 g (corresponding to 3.67 mmol) of 6-promo-2-[4'-(3''-hydroxypropoxy)phenyl]imidazo[1,2-a]pyridine (Fig. 1-2, Step 3).

177 mg (corresponding to 0.5 mmol) of 6-bromo-2-[4'-(3''-hydroxypropoxy)phenyl]imidazo[1,2-a]pyridine dissolved in 10 mL of pyridine, and 197 mg (corresponding to 1.0 mmol) of p-tolueneaulfonylchloride was added under an ice bath. After the reaction solution was stirred at room temperature for 16 hours, it was poured into water and extracted three times with chloroform. The combined chloroform layer was washed with a saturated sodium chloride solution, dried over anhydrous sodium sulfate, filtered and concentrated, The resulting crude product was purified by recycle preparative HPLC (HPLC apparatus: LC-908 (under trade name: manufactured by Japan Analytical Industry Co., Ltd.); column: two JAIGEL 2H (under trade name; manufactured by Japan Analytical Industry Co., Ltd.) connected together; mobile phase: chloroform), to obtain 87 mg (corresponding to 0.17 mmol) of 6-bromo-2-[4'-(3''-p-toluenesulfonyloxypropoxy)phenyl]imidazo[1,2-a]pyridine (Fig. 1-2, Step 4).

The NMR measurement results of the resulting 6-bromo-2-[4'-(3''-p-toluenesulfonyloxypropoxy)phehyl]imidazo [1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): δ 8.26-8.24 (m, 1H), 7.84-1.80 (m, 2H), 7.77-7.74 (m, 2H), 7.74 (s, 1H), 7.50 (d, J = 9.7 Hz, 1H), 7.26-7.23 (m, 2H), 7.21 (dd, J = 9.7, 2.0 Hz, 1H), 6.84-6.80 (m, 2H), 4.26 (t, J = 6.0 Hz, 2H), 3.98 (t, J = 6.0 Hz, 2H), 2.35 (s, 3H), 2.13 (quint., J = 6.0 Hz., 2H).

$^{13}$C-NMR (solvent: chlorororm-dl, resonance frequency: 125 MHz): δ 159.67, 146.53, 144.79, 144.08, 132.77, 129.80, 127.87, 127.81, 127.28, 126.20, 125.43, 117.87, 114.63, 107.40, 106.76, 66.97, 63.08, 28.85, 21.60.

Example I-5: Synthesis of 6-bromo-2- [4'- (3''-[$^{18}$F]fluoropropoxy)phenyl]imidazo[1,2-a]pyridine

[$^{19}$F]fluoride ion-containihg $H_2{}^{18}O$ (radioactivity: 4210 MBq, a value converted at the beginning of synthesis) was parsed through a Sep-Pak Light QMA (under trade name; manufactured by Japan Waters K.K.) to absorb and collect [$^{18}$F]fluoride ions. Then, a potassium carbonate solution (66.7 mmol/L, 0.3 mL) and 1.5 mL of a solution of 20 mg (corresponding to 53.2 μmol) of Kryptofix 222 (under trade name; manufactured by Merck Co., Ltd.) in acetonitrile were passed through the column to elute the [$^{18}$F]fluoride ions.

The eluate was heated under helium gas stream to 100°C to evaporate water, and supplemented with actetonitrile (0.3 mL×2) and azeotropically distilled to dryness. To this, 1.0 mL of a solution of 5 mg (corresponding to 10.0 μmol) of 6-bromo-2-[4'-(3''-p-touleilesulfonyloxypropoxy)phenyl]imidazo[1,2-a]pyridine synthesized above in Example I-4 in dimethylformamide was added thereto, and heated at 130°C for 10 minutes. After the reaction solution was cooled down to 30°C, it was passed through a Sep-Pak Plus Silica (trade lame ; Japan Waters K.K.) each time the reaction solution was supplemented with ether (3.5 mL x 3). The ether solution that had been passed was heated to 60°C under helium gas stream and concentrated. The concentrated solution was diluted with 2 mL of a mixed solution of methanol/water/triethyamine = 800:200:1.

The resulting solution was purified by HPLC (column: Capcell Pak C18 MG (15 mm i.d. x 250 mm, manufactured by Shiseido Co., Ltd.); elution solvent: methanol/water/triethylamine = 7007300/1), An eluate fraction containing the target compound is diluted witch 100 mL of water, and then passed through a Sep-Pak Plus C18 (trade name, manufactured by Japan Waters K.K.) to adsorb and collect the target compound. Then, 20 mL of water was passed through the column to wash it. Then, 4 mL of ethanol was passed through the column to elute a solution of 6-bromo-2-[4'-(3''-[$^{18}$F] fluoropropoxy)phenyl]imidazo[1,2-a]pyridine in ethanol. The obtained radioactivity level was 769 MBq (107 min. after the beginning of synthesis). According to the TLC analysis on the following conditions, the radiochemical purity thereof was 95.9%.

TLC analysis conditions:

TLC plate: Silica Gel 60 $F_{254}$ (trade name; manufactured by Merck & Co., Inc.)
Mobile phase: Chloroform/methanol/triethylamine = 500/10/0.5
Detector: Rita Star (trade name; manufactured by raytest)

Example 1-6: Synthesis of 6-bromo-2-[4'-(2''-p-toluenesulfonyloxyethoxy)phenyl]imidazo[1,2-a]pyridine

50 mL of ethyl acetate was added to 28.17 g (corresponding to 126 mmol) of cupric bromide to obtain a suspension, to which a solution, of 8.18 g (corresponding to 60.0 mmol) of 4'-hydroxyacetophenone in a mixed solution of 50 mL of ethyl acetate and 50 mL of chloroform was added. Then, the resulting mixture was reflexed. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified bey flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 1-3, Step 1).

2.15 g (corresponding to 10.0 mmol) of 2-bromo-4'-hydroxyacetophenone and 1.74 g (corresponding to 10.0 mmol) of 2-amino-5-bromopyridine were dissolved in 50 mL of acetonitrile. The resulting solution was refluxed in an oil bath at 105°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 2.41 g (corresponding to 8.32 mmol) of 6-bromo-2-(4'-hydroxyphenyl)imidazo[1,2-a]pyridine (Fig. 1-3, Step 2).

621 mg (corresponding to 10.0 mmol) of ethylene glycol was dissolved in 100 mL of methylene chloride. To this solution under an ice bath, 3.49 g (corresponding to 15.0 mmol) of silver oxide, 350 mg (corresponding to 2.1 mmol) of potassium iodide and 2.10 g (corresponding to 11.0 mmol) of p-toluenesulfonylchloride were added. The resulting mixture was stirred at 0°C for 2 hours. Insoluble matters were filtered out of the reaction mixture, and were washed with ethyl acetate. The washings were combined with the filtrate, and the mixture was concentrated. The resulting crude product was purified by flash silica gel column chromatography (elution solvent: hexane/ethyl acetate = 1/1) to obtain 643 mg (corresponding to 2.97 mmol) of 2-hydroxyethyl-p-toluenesulfonate (Fig. 1-3, Step3).

To 10 mL of a solution of 639 mg (corresponding to 2.95 mmol) of 2-hydroxyethyl-p-toluenesulfonate in tetrahydrofran, 388 mg (corresponding to 1.34 mmol) of 6-bromo-2-(4'-hydroxyphenyl)imidazo[1,2-a]pyridine and 780 mg (corresponding to 2.97 mmol) of triphenylphosphine were added. Further, 6 mL of N,N-dimethylformamide was added thereto to completely dissolve the contents. To the reaction mixture 0.58 mL (corresponding to 2.95 mmol) of diisopropylazodicarboxylate was added. After the reaction mixture was stirred at room temperature for 17 hours, the reaction solution was concentrated. The resulting crude product was purified by flash silica gel column chromatography (elution solvent: hexane/ethyl acetate = 65/35). Insoluble matter in chloroform was filtered out of fractions containing a target compound. Further, the resulting crude product was purified by recycle preparative HPLC (HPLG apparatus: LC-908 (under trade name; manufactured by Japan Analytical Industry Co., Ltd.); column: two JAIGEL 2H (under trade name; manufactured by Japan Analytical Industry Co., Ltd.) connected together; mobile phase: chloroform), to obtain 79.7 mg (corresponding to 164 $\mu$mol) of 6-bromo-2-[4'-(2''-p-toluenesulfonyloxyethoxy)phenyl]imidazo[1,2-a]pyridine (Fig. 1-3, Step 4).

The NMR measurement results of the resulting 6-bromo-2-[4'-(2''-p-toluenesulfonyloxyethoxy)phenyl]imidazo [1,2-a]pyridine are shown bellow.

NMR apparatus employed: JNM-ECP-500 (manufactured by Japan Electron Optics Laboratory Co., Ltd, (JEOL)) $^1$H-NMR (solvent: dimethylformamide-d7, resonance frequency: 500 MHz): $\delta$ 8.73-8.71 (m, 1H), 8.19-8.17 (m, H), 7.81-7.77 (m, 2H), 7.73-7.70 (m, 2H), 7.41-7.38 (m, 1H), 7.39-7.36 (m, 2H), 7.20 (dd, J = 9.5, 1.9 Hz), 6.85-6.81 (m, 2H), 4.34-4.31 (m, 2H), 4.19-4.15 (m, 2H).

$^{13}$C-NMR (solvent: dimethylformamide-d7, resonance frequency: 125 MHz): $\delta$ 158.32, 145.91, 145.24, 143.84, 133.15, 130.18, 127.83, 127.54, 127.19, 127.15, 126.90, 117.56, 114.86, 108.73, 105.80, 69.28, 65.88, 20,69,

Reference Example I-1: Synthesis of 6-bromo-2-[4'-(3''-fluoropropoxy)phenyl]imidazo[1,2-a]pyridine (non-radioactive fluorinated form)

As a sample for evaluating affinity with amyloid, Solubility in fat and mutagenicity of the present compound, a non-radioactive fluorinated form of 6-bromo-2-[4'-(3''-fluoropropoxyphenyl)imidazo[1,2-a]pyridine was synthesized.

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction, mixture was cooled down to room temperature and filtered. The resulting filtrate was concentrated under reduced pressure. The residue was dissolved in ethyl acetate and subjected to decoloring operation with addition of

active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7,25 g (corresponding to 33.7 mmol) of 2-bromo-4'-hydroxyacetophenone (Fig. 1-4, step 1).

2.15 g (corresponding to 10.0 mmol) of 2-bromo-4'-hydroxyacetophenone and 1.74 g (corresponding to 10.0 mmol) of 2-amino-5-bromopyridine were dissolved in 50 mL of acetonitrile. The resulting solution was refluxed in an oil bath at 105˚C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture was sonicated for 5 minutes using an ultrasonic washing machine. for 5 minutes using an ultrasonic washing machine. Precipitates were filtered and recovered from the resulting mixture, sufficiently washed with water, and dried under reduced pressure, to obtain 2.41 g (corresponding to 8.32 minos) of 6-bromo-2-(4'-hydroxyphenyl)imidazo[1,2-a]pyridine (Fig. 1-4, Step 2).

290 mg (corresponding to 1.0 mmol) of 6-bromo-2-(4'-hydroxyphenyl)imidazo[1,2-a]pyridine that was sufficiently dried to remove moisture was dissolved in 10 mL of N,N-dimethylformamide, and 413 mg (corresponding to 3.0 mmol) of potassium carbonate was added thereto. The mixture was supplemented with 138 $\mu$L (corresponding to 1.5 mmol) of 1-bromo-3-fluoroprppahe, and then was stirred at room temperature for 20.5 hours. After the completion of the reaction, the reaction solution was poured into water and extracted three times with chloroform. The combined chloroform layer was washed with a saturated sodium chloride solution, dried over anhydrous sodium sulfate, filtered and concentrated. The resulting crude product was purified by recycle preparative HPLC (HPLC apparatus: LC-908 (under trade name; manufactured by Japan Analytical Industry Co., Ltd.); column: two JAIGEL 2H (under trade name; manufactured by Japan Analytical Industry Co., Ltd.) connected together; mobile phase: chloroform), to obtain 302 mg (corresponding to 0.866 mmol) of 6-bromo-2-[4'-(3''-fluoropropoxy)phenyl]imidazo[1,2-a]pyridine (Fig. 1-4, Step 3).

The NMR measurement results of the resulting 6'-bromo-2-[4'-(3''-fluoropropoxy)phenyl]imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): $\delta$ 8.23 (dd, J = 1.9, 0.2 Hz, 1H), 7,88-7.83 (m, 2H), 7.51-7.48 (m, 1H), 8.21 (dd, J = 9.5, 1.9 Hz, 1H), 6.99-6.95 (m, 2H), 4.67 (dt, $^2J_{HF}$ = 47.1 Hz, J = 5.9 Hz, 2H), 4.15 (t, J = 5,9 Hz, 2H), 2.19 (dquint, $^3J_{HF}$ = 25.9 Hz, J = 5.9 Hz, 2H).

$^{13}$C-NMR (solvent: chloroform-dl, resonance frequency; 125 MHz): $\delta$ 159,01, 146.61, 144.07, 127.81, 127.38, 126.15, 125.41, 117.87, 114.78, 107.41, 106.71, 80.71 (d, $^1J_{CF}$ = 164.6 Hz), 63.59 (d, $^3J_{CF}$ = 5.3 Hz), 30.43 (d, $^2J_{CF}$ = 19.7 Hz).

$^{19}$F-NMR (solvent: chloroform-dl, resonance frequency: 470 MHz) : $\delta$ -222.07 (dd, $^2J_{HF}$ = 47.1 Hz, $^3J_{HF}$ = 25.9 Hz).

Reference Example I-2: Synthesis of 2-[4'-(3''-fluoropropoxy)phenyl]-6-iodoimidazo[1,2-a]pyridine (non-radioactive fluorinated form)

: As a sample for evaluating affinity with amyloid, solubility in fat and mutagenicity of the present compounds, a non-radioactive fluorinated form of 2-[4'-(3''-fluoropropoxy)phenyl]-6-iodoimidazo[1,2-a]pyridine was synthesized.

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvents chloroform/methanol = 20/1), and recrystallized from ethyl acetate/petroleum ether, to obtain 7.25 g (corresponding to 33.7 mmol) of 2-bromo-4'-hydroxyacetophenone (Fig. 1-5, Step 1).

441 mg (corresponding to 2.0 mmol) of 2-bromo-4'-hydroxyacetophenone and 449 mg (corresponding to 2.0 mmol) of 2-amino-5-iodopyridine were dissolved in 15 mL of acetonitrile. The resulting solution was refluxes in an oil bath at 110˚: for 5 hours. After the completion of the reaction, the reaction solution was cooled down to room temperature, and precipitates were filtered and recovered. The precipitates were washed, with acetonitrile and dried under reduced pressure. The resulting crude crystals were suspended in a mixed solution of 10 mL of water and 10 mL of methanol. Then, about 10 mL of a saturated sodium hydrogencarbonate solution was added thereto, and the mixture was sonicated for minutes using an ultrasonic washing machine. Precipitates were: filtered and recovered from the resulting fixture, sufficiently washed with water, and dried under reduced pressure, to obtain 526 mg (corresponding to 1.56 mmol) of 2-(4'-hydroxyphenyl)-6-iodoimidazo[1,2-a]pyridine (Fig. 1-5, Step 2).

673 mg (corresponding to 2.0mmol) of 2-(4'-hydroxyphenyl)-6-iodoimidazoio[1,2-a]pyridine that was sufficiently dried to remove moisture was dissolved in 25 mL of N,N-dimethylformamide, and 831 mg (corresponding to 6.0 mmol) of potassium carbonate was added thereto. The mixture was supplemented with 275 μL (corresponding to 3.0 mmol) of 1-bromo-3-fluoropropane, and then stirred at room temperature for 24 hours. After the completion of the reaction, the reaction solution was poured into water and extracted three times with chloroform. The combined chloroform layer was washed with water and a saturated sodium chloride solution, dried over anhydrous sodium sulfate, filtered and concentrated. The resulting crude product was purified by flash silica gel column chromatography (elution solvent: chloroform), and further by recycle preparative HPLC (HPLC apparatus: LC-908 (under trade name; manufactured by Japan Analytical Industry Co., Lid.); column: two JAIGEL 2H (under trade name; manufactured by Japan Analytical Industry Co., Ltd.) connected together; mobile phase: chloroform), to obtain 349 mg (corresponding to 0,881. mmol) of 2-[4'-(3''-fldoropropoxy)phenyl]-6'-iodoimidazo[1,2-a]pyridine (Fig. 1-5, Step 3).

The NMR measurement results of the resulting 2-[4'-(3''-fluoropropoxy)phenyl]-6-iodoimidazo[1,2-a]pyridine (internal standard: tetramethylsilane) are shown, below.

NMR apparatus employed: JNM-ECP-500 (manufactured by Japan Electro Optics Laboratory Co., Ltd. (JEOL)) $^1$H-NMR (solvent: chloroform-dl, resonance frequency: 500 MHz): δ 8,37-8.35 (m, 1H), 7.38-7.84 (m, 2H), 7.72 (s, 1H), 7.42-7.39 (m, 1H), 7.32 (dd, J = 9.14., 1.6 Hz, 1H), 6.99-6.96 (m, 2H), 4.67 (dt, $^2J_{HF}$ = 47.0 Hz, J = 6.0 Hz, $^2$H), 4.15 (t, J = 6.0 Hz, 2H), 2.20 (dquint, $^3J_{HF}$ = 25.9 Hz, J = 6.0 Hz, 2H).

$^{13}$C-NMR (solvent: chloroform-dl, resonance frequency: 125 MHz) : δ 159.01, 146.23, 144.16, 132.36, 130.28, 127.42, 126.05, 118.31, 114.77, 106.90, 80.72 (d, $^1J_{CF}$ = 164.6 Hz), 74.80, 63,57 (d, $^3J_{CF}$ = 5.3 Hz), 30.42 (d, $^2J_{CF}$ = 20.2 Hz).

$^{19}$F-NMR (solvent: chloroform-dl, resonance frequency: 470 MHz): δ -222.09 (dd, $^2J_{HF}$ = 47.0 Hz, $^3J_{HF}$ = 25.9 Hz),

<u>Reference Example 1-3: Synthesis of 6-bromo-2-[4'-(2''-fluoroethoxy)phenyl]imidazo[1,2-a]pyridine (non-radioactive fluorinated form)</u>

As a sample for evaluating affinity with amyloid, Solubility in fat and mutagenicity of the present compounds, a non-radiocactive fluorinated form of 6-promo-2-[4'-(2''-fluoroethoxy)phenyl]imidazo[1,2-a]pyridine was synthesized.

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.2.5 g (corresponding to 33.7 mmol) of 2-bromo-4'-hydroxyacetophenone (Fig. 1-6, Step 1).

2.15 g (corresponding to 10.0 mmol) of 2-bromo-4'-hydroxyacetophenone and 1.74 g (corresponding to 10.0 mmol) of 2-amino-5-bromopyridine were dissolved in 50 mL of acetonitrile. The resulting solution was refluxed in an oil bath at 105°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and resulting mixture, sufficiently washed with water, and dried under reduced pressure, to obtain 2.41 g (corresponding to 8.32 mmol) of 6-bromo-2-(4'-hydroxyphenyl)imidazo[1,2-a]pyridine (Fig. 1-6, Step 2).

578 mg (corresponding to 2.0 mmol) of 6-bromo-2-(4'-hydroxyphenyl)imidazo[1,2-a]pyridine that was sufficiently dried to remove moisture was dissolved in 20 mL of N,N-dimethylformamide, and 830 mg (corresponding to 6.0 mmol) of potassium carbonate was added thereto. The mixture was supplemented with 510 μL (corresponding to 3.0 mmol) of 2-fluoroethyl-p-toluenesulfonate, and then the solution was stirred at room temperature for 44.5 hours. After the completion of the reaction, the reaction solution was poured into water and extracted three times with chloroform. The combined chloroform layer was washed with water and a saturated sodium chloride solution, dried over anhydrous sodium sulfate, filtered and concentrated. The resulting crude product was purified by flash silica gel column chromatography (elution solvent: chloroform/methanol = 100/1), by recycle preparative HPLC (HPLC apparatus: LC-908 (under trade name; manufactured by Japan Analytical Industry Co., Ltd.); column: two JAIGEL 2H (under trade name; manufactured by Japan Analytical Industry Co., Ltd.) connected together; mobile phase: chloroform), and further by preparative TLC (elution solvent: chloroform/methanol = 50:1) to obtain 446 mg (corresponding to 1.33 mmol) of 6-bromo-2-[4'-(2''-fluoroethoxy)phenyl]imidazo[1,2-a]pyridine (Fig. 1-6, Step 3).

The NMR measurement results of the resulting 6-bromo-2-[4'-[2''-fluoroethoxy)phenyl]imidazo[1,2-alpyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): δ 8.23-8.21 (m, 1H), 7.87-7.84 (m, 1H), 7.72 (s, 1H), 7.51-7.47 (m, 1H), 7.20 (dd, J = 9.5, 1.9 Hz, 1H), 7.01-6.97 (m, 2H), 4.84-4.71 (m, 2H), 4.30-4.21 (m, 2H).

$^{13}$C-NMR (solvent: chloroform-dl, resonance frequency: 125 MHz): δ 1.58.62, 146.46, 144.06, 127.85, 127.41, 126.58, 125.42, 117.87, 114.91, 107.49, 106.74, 81.86 (d, $^1J_{CF}$ = 170.8 Hz), 67.15 (d, $^2J_{CF}$ = 20.2 Hz).

$^{19}$F-NMR (solvent: chloroform-dl, resonance frequency: 470 MHz): δ -223.80 (dd, $^2J_{HF}$ = 47.4 Hz, $^3J_{HF}$ = 27.6 Hz).

<u>Reference Example I-4: Synthesis of 2-[4'-(2''-fluoroethoxy)phenyl]-6-iodoimidazo[1,2-a]pyridine (non-radioactive fluorinated form)</u>

As a sample for evaluating affinity with amyloid, solubility in fat and mutagenicity of the present compounds, a non-radioactive fluorinated form of 2-[4'-(2''-fluoroethoxy)phenyl]-6-iodoimidazo[1,2-a]pyridine was synthesized.

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 1-7, Step 1).

441 mg (corresponding to 2.0 mmol) of 2-bromo-4'-hydroxyacetophenone and 449 mg (corresponding to 2.0 mmol) of 2-amino-5-iodopyridine were dissolved in 15 mL of acetonitrile. The resulting solution was refluxed in of acetonitrile. The resulting solution was refluxed in an oil bath at 110°C for 5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 10 mL of methanol. Then, about 10 mL of a saturated sodium hydrogencarbonate solution was added thereto, and the mixture was sonicated for 5 Minutes using an ultrasonic washing machine. Precipitates were filtered and recovered from the resulting mixture, sufficiently washed with water, and dried under reduced pressure, to obtain 526 mg (corresponding to 1.56 mmol) of 2-(4'-hydroxyphenyl)-6-iodoimidazo[1,2-a]pyridine (Fig. 1-7, Step 2).

368 mg (corresponding to 1.1 mmol) of 2-(4'-hydroxyphenyl)-6-iodoimidazo[1,2-a]pyridine that was sufficiently dried to remove moisture was dissolved in 15 mL of N,N-dimethylformamide, and 453 mg (corresponding to 3.3 mmol) of potassium carbonate was added thereto. The mixture was supplemented with 280 μL (corresponding to 1.6 mmol) of 2-fluoroethyl-p-toluenesulfonate, and then the solution was stirred at room temperature for 22 hours. After the completion of the reaction, the reaction solution was poured into water and extracted three times with chloroform. The combined chloroform layer was washed with water and a saturated sodium chloride solution, dried over anhydrous sodium sulfate, filtered and concentrated. The resulting crude product was purified by flash silica gel column chromatography (elution solvent: hexane/ethyl acetate = 1/1), and further by recycle preparative HPLC (HPLC apparatus: LC-908 (under trade name; manufactured by Japan Analytical Industry Co., Ltd.); column: two JAIGEL 2H (under trade name; manufactured by Japan Analytical Industry Co., Ltd.) connected together; mobile phase: chloroform), to obtain 222 mg (corresponding to 0.580 mmol) of 2-[4'-(2''-fluoroethoxy)phenyl]-6-iodoimidazo[1,2-a]pyridine (Fig. 1-7, Step 3).

The NMR measurement results of the resulting 2-[4'-(2''-fluoroethoxy)phenyl]-6-iodoimidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): δ 8.35-8.33 (m, 1H), 7.88-7.84 (m, 2H), 7.70 (s, 1H), 7.39 (d, J = 9.4 Hz, 1H), 7.31 (dd, J = 9.4, 1.8 Hz, 1H), 7.01-6.97 (m, 2H), 4.84-4.71 (m, 2H), 4.30-4.22 (m, 2H).

$^{13}$C-NMR (solvent: chloroform-dl, resonance frequency: 125 MHz): δ 158. 62, 146.08, 144.16, 132.38, 130.30, 127.44, 126.52, 118.30, 114.91, 106.99, 81.86 (d, $^2J_{CF}$ = 170.8 Hz), 74.82, 67.15 (d, $^3J_{CF}$ = 20.6 Hz).

$^{19}$F-NMR (solvent: chloroform-dl, resonance frequency: 470 MHz): δ -223.74 (dd, $^2J_{HF}$ = 47.4 Hz, $^3J_{HF}$ = 27.7 Hz).

Reference Example I-5: Synthesis of 2-[4'-(3"-fluoropropoxy)phenyl]-6-iodoimidazo[1,2-a]pyrimidine (non-radioactive fluorinated form)

As a sample for evaluating affinity with amyloid, solubility in fat and mutagenicity of the present compounds, a non-radioactive fluorinated form of 2-[4'-(3"-fluoropropoxy)phenyl]-6-iodoimidazo[1,2-a]pyridine was synthesized.

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 1-8, Step 1).

646 mg (corresponding to 3.0 mmol) of 2-bromo-4'-hydroxyacetophenone and 668 mg (corresponding to 3.0 mmol) of 2-amino-5-iodopyrimidine were dissolved in 20 mL of acetonitrile. The resulting solution was refluxed in an oil bath at 110°C for 8 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 10 mL of methanol. Then, about 15 mL of a saturated sodium hydrogencarbonate solution was added thereto, and the mixture was sonicated for 3 minutes using an ultrasonic washing machine. Precipitates were filtered and recovered from the resulting mixture, sufficiently washed with water, and dried under reduced pressure, to obtain 737 mg (corresponding to 2.19 mmol) of 2-(4'-hydroxyphenyl)-6-iodoimidazo[1,2-a]pyrimidine (Fig. 1-8, Step 2).

339 mg (corresponding to 1.0 mmol) of 2-(4'-hydroxyphenyl)-6-iodoimidazo[1,2-a]pyrimidine that was sufficiently dried to remove moisture was dissolved in 20 mL of N,N-dimethylformamide, and 414 mg (corresponding to 3.0 mmol) of potassium carbonate was added thereto. The mixture was supplemented with 138 μL (corresponding to 1.5 mmol) of 1-bromo-3-fluoropropane, and then stirred at room temperature for 22 hours. After the completion of the reaction, the reaction solution was poured into water and extracted three times with chloroform. The combined chloroform layer was washed with a saturated sodium chloride solution, dried over anhydrous sodium sulfate, filtered and concentrated. The resulting crude product was recrystallized from N,N-dimethylformamide to obtain 236 mg (corresponding to 0.594 mmol) of 2-[4'-(3"-fluoropropoxy)phenyl]-6-iodoimidazo[1,2-a]pyrimidine (Fig. 1-8, Step 3).

The NMR measurement results of the resulting 2-[4'-(3"-fluoropropoxy)phenyl]-6-iodoimidazo[1,2-a]pyrimidine (internal standard: dimethylsulfoxide) are shown below.

NMR apparatus employed: JNM-ECP-500 (manufactured by Japan Electron Optics Laboratory Co., Ltd. (JEOL)) $^1$H-NMR (solvent: dimethylsulfoxide-d6, resonance frequency: 500 MHz): δ 9.27 (d, J = 2.3 Hz, 1H), 8.55 (d, J = 2.3 Hz, 1H), 8.15 (s, 1H), 7.94-7.90 (m, 2H), 7.06-7.02 (m, 2H), 4.62 (dt, $^2J_{HF}$ = 47.2 Hz, J = 6.1, 2H), 4.14 (t, J = 6.1 Hz, 2H), 2.13 (dquint, $^3J_{HF}$ = 25.5 Hz, J = 6.1 Hz, 2H).

$^{13}$C-NMR (solvent: dimethylsulfoxide-d6, resonance frequency: 125 MHz): δ 159.16, 154.12, 146.54, 146.26, 139.00, 127.60, 126.06, 115.21, 106.52, 81.15 (d, $^1J_{CF}$ = 161.7 Hz), 74.43, 64.07 (d, $^3J_{CF}$ = 5.8 Hz), 30.13 (d, $^2J_{CF}$ = 19.7 Hz).

$^{19}$F-NMR (solvent: dimethylsulfoxide-d6, resonance frequency: 470 MHz): δ -220.13 (tt, $^2J_{HF}$ = 47.2 Hz, $^3J_{HF}$ = 25.5 Hz).

Reference Example I-6: Synthesis of [$^{125}$I]-IMPY

[$^{125}$I]-IMPY was synthesized in accordance with the following steps for use in Comparative Examples for evaluation on binding to amyloid

In accordance with the method described in a literature (Zhi-Ping Zhuang et al., J. Med. Chem, 2003, 46, p. 237-243), 6-tributylstannyl-2-[4'-(N,N-dimethylamino)phenyl]imidazo[1,2-a]pyridine was synthesized, and dissolved in methanol (concentration: 1mg/mL). To 53 μL of the resulting solution, 75 μL of 1 mol/L hydrochloric acid, 20 μL of [$^{125}$I] sodium iodide of 13.5 MBq and 10 μL of 10% (w/v) hydrogen peroxide were added. After the mixed solution was left to stand at 50°C for 10 minutes, the solution was subjected to HPLC under the same condition as described in Example I-2, to obtain [$^{125}$I]-IMPY fraction.

10 ml of water was added to the fraction. The resulting solution was passed through a reverse phase column (trade name: Sep-Pak (registered trademark) Light C18 Cartridges manufactured by Waters; the packed amount of the packing agent: 130 mg), so that the column adsorbs and collects the [$^{125}$I]-IMPY. The column was rinsed with 1 mL of water, and then 1 mL of ethanol was passed therethrough, to elute [$^{125}$I]-IMPY. The obtained radioactivity was 2.6 MBq at the end of synthesis. Further, the TLC analysis was conducted under the same conditions as described in Example

I-2, and as a result, the radiochemical purity of the compound was 98.0%.

Reference Example I-7: Synthesis of [$^{123}$I]-IMPY

[$^{123}$I]-IMPY was prepared in accordance with the following steps for use in Comparative Examples for evaluations on $logP_{octanol}$ and accumulation in brain.

In accordance with the method described in a literature (Zhi-Ping Zhuang et al., J. Med. Chem., 2003, 46, p. 237-243), 6-tributylstannyl-2-[4'-(N,N-dimethylamino)phenyl]imidazo[1,2-a]pyridine was synthesized, and dissolved it methanol (concentration: 1mg/mL). To 53 μL of the resulting solution, 100 μL of 1 mol/L hydrochloric acid, 20-50 μL of [$^{123}$I]sodium iodide of 190-240 MBq, 10 μL of 1 mmol/L sodium iodide and 10 μL of 10% (w/v) hydrogen peroxide were added. After the mixed solution was left to stand at 50°C for 10 minutes, the solution was subjected to HPLC under the same condition as described in Example I-2, to obtain [$^{123}$I]-IMPY fraction.

10 ml of water was added to the fraction. The resulting solution was passed through a reverse phase column (trade name: Sep-Pak (registered trademark) Light C18 Cartridges manufactured by Waters; the packed amount of the packing agent: 130 mg), so that the column adsorbs and collects the [$^{123}$]-IMPY. The column was rinsed with 1 mL of water, and then 1 mL of ethanol was passed therethrough, to elute [$^{123}$I]-IMPY. The obtained radioactivity was 47-56 MBq at the end of synthesis. Further, the TLC analysis was conducted under the same conditions as described in Example I-2, and as a result, the radiochemical purity of the compound was 98.0%.

Reference Example I-8: Synthesis of 2-(4'-hydroxyphenyl)-6-[$^{125}$I]iodoimidazo[1,2-a]pyridine

In order to prepare calculation formulas for the calculation of $logP_{HPLC}$, 2-(4'-hydroxyphenyl)-6-[$^{125}$I]iodoimidazo [1,2-a]pyridine was synthesized in accordance with the following steps.

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated under reduced pressure. The residue was dissolved in ethyl acetate and subjected to decoloring operation, with addition of active charcoal. Then, the resulting solution was filtered and concentrated. The resulting crude product was purified by flash silica gel column chromatography (elution solvent: cHloroform/methanol = 20/1), and recrystallized from ethyl acetate/petroleum ether, to obtain 7.25 g (corresponding to 33.7 mmol) of 2-bromo-4'-hydroxyacetophenone (Fig. 1-9, Step 1).

2.15 g (corresponding to 10.0 mmol) of 2-bromo-4'-hydroxyacetophenone and 1.74 g (corresponding to 10.0 mmol) of 2-amino-5-bromopyridine were dissolved in 50 mL of acetonitrile. The resulting solution was refluxed in an oil bath at 105°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture was sonicated for 5 minutes using an ultrasonic washing machine. Precipitates were faltered and recovered from the resulting mixture, sufficiently washed with water, and dried under reduced pressure, to obtain 2.41 g (corresponding to 8.32 mmol) of 6-bromo-2-(4'-hydroxyphenyl)imidazo[1,2-a]pyridine (Fig. 1-9, Step 2).

138 mg (corresponding to 0.476 mmol) of 6-promo-2-(4'-hydroxyphenyl)imidazo[1,2-a]pyridine was dissolved in 20 mL of dioxane, and 2 mL of triethylamine was added thereto. Then, 360 μL (corresponding to 0.713 mmol) of bis (tributyltin) and 20 mg (a catalytic amount) of tetrakis-triphenylphosphine palladium were added thereto. After the reaction mixture was stirred at 90°C for 22 hours, the solvent was distilled off under reduced pressure. The residue was purified by preparative TLC (elution solvent: hexane/ethyl acetate = 1/4). Further, the resulting crude product was purified by recycle preparative HPLC (HPLC apparatus: LC-908 (under trade name: manufactured by Japan Analytical Industry Co., Ltd.); column: two JAIGEL 2H (under trade name; manufactured by Japan Analytical Industry Co., Ltd.) connected together; mobile phase; chloroform), to obtain 47 mg (corresponding to 94.9 μmol) of 6-tributylstannyl-2-(4'-hydroxyphenyl)imidazo[1,2-a]pyridine (Fig. 1-9, Step 3).

To 53 μL of a solution of 6-tributylstannyl-2-(4'-hydroxyphenyl)imidazo[1,2-a]pyridine in methanol (concentration: 1 mg/mL), 75 μL of 1 mol/L hydrochloric acid, 40 μL of [$^{125}$I]sodium iodide of 136 MBq and 10 μL of 10% (w/v) hydrogen peroxide were added. After the mixed solution was left to stand at 50°C for 10 minutes, the solution was subjected to HPLC under the same condition as in Example 1-2, to obtain 2-(4'-hydroxyphenyl)-6-[$^{126}$I]iodoimidazo[1,2-a]pyridine fraction (Fig. 1-9, Step 4).

10 ml of water was added to the fraction. The resulting solution was passed through a reversed phase column (trade name: Sep-Pak (registered trademark) Light C18 Cartridges manufactured by Waters: the packed amount of the packing agent: 130 mg) so that the column adsorbs and collects 2-(4'-hydroxyphenyl)-6-[$^{125}$I]iodoimidazo[1,2-a]pyridine.

The column was rinsed witch 1 mL of water, and then 1 mL of ethanol was passed therethrough, to elute 2-(4'-hydroxyphenyl)-6-[$^{125}$I]iodoimidazo[1,2-a]pyridine. The obtained radioactivity was 37.5 MBq at the end of synthesis. Further, the TLC analysis was conducted under the same conditions as in Example I-2, and as a result, the radiochemical purity of the compound was 96.5%.

Reference Example I-9: Synthesis of 2-(4'-hydroxyphenyl)-6-iodoimidazo[1,2-a]pyridine

50 mL of ethyl acetate was added to 28.17 g (corresponding to 126 mmol) of cupric bromide to obtain a suspension to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 1-10, Step 1).

441 mg (corresponding to 2.0 mmol) of 2-bromo-4'-hydroxyacetophenone and 449 mg (corresponding to 2.0 mmol) of 2-amino-5-iodopyidine were dissolved in 15 mL of acetonitrile. The resulting solution was refluxed in an oil bath at 110˚C for 5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 10 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting fixture, sufficiently washed with water, and dried under reduced pressure, to obtain 526 mg (corresponding to 1.56 mmol) of 2-(4'-hydroxyphenyl)-6-iodoimidazo[1,2-a]pyridine (Fig. 1-10, Step 2).

The NMR measurement results of the resulting 2-(4'-hydroxyphenyl)-6-iodoimidazo[1,2-a]pyridine (internal standard: dimethylsulfoxide) are shown below.

NMR apparatus employed: JNM-ECP-500 (manufactured by Japan Electron Optics Laboratory Co., Ltd. (JEOL)) $^1$H-NMR (solvent: dimethylsulfoxide-d6, resonance frequency: 500 MHz): δ 8.86-3.84 (m, 1H), 8.14 (s, 1H), 7.73-7.74 (m, 2H), 7.40-7.35 (m, 2H), 6.86-6.82 (m, 2H).

$^{13}$C-NMR (solvent: dimethylsulfoxide-d6, resonance frequency: 125 MHz): δ 158.08, 145.87, 143.87, 132.48, 131.72, 127.67, 124.99, 118.14, 116.14, 108.02, 75.85.

Examples I-7 and Comparative Examples I-1: Measurement of binding to amyloid

Affinity of the present compounds with amyloid was examined by the following *in vitro* binding tests.

(1) A$\beta_{1-40}$ (Peptide Institute, INC.) was dissolved in phosphate buffer (pH 7.4) and shaken at 37˚C for 72 hours, to obtain a 1 mg/mL suspension (hereinafter referred to as amyloid suspension in these Examples) of aggregated Aβ (hereinafter referred to as amyloid in this Example).

(2) According to the method described in a literature (Naiki, H., et al., Laboratory Investigation 74, p.374-383 (1996)), the amyloid suspension was subjected to qualitative experiment based on fluorescence spectrophotometric method using Thioflavin T (manufactured by Fluka) to confirm that the aggregated Aβ obtained in (1) was amyloid (measurement conditions: excitation wavelength of 446 nm, and emission wavelength of 490 nm).

(3) A solution in ethanol of the compound I-6 synthesized by a method described above in Example I-2 (radioactive concentration: 37 MBq/mL) was prepared, and diluted with a 0.1 % bovine serum albumin-containing phosphate buffer (pH 7.4) to prepare a solution corresponding to 2 nmol/L as a total amount of 2-[4'-(3''-fluoropropoxy)phenyl]-6-iodoimidazo[1,2-a]pyridine.

4) To each well of a 96-well microplate, 50 μL of a solution (400 pM at a final concentration) prepared above in (3) and 50 μL of a solution (amyloid concentration may be adjusted in accordance with amyloid concentration in a sample solution) where amyloid suspension was dissolved in a 0.1 % bovine serum albumin-containing phosphate buffer (pH 7.4), were added, and then 150 μL of the same buffer was added to prepare a solution of amyloid at final concentrations of 2.5, 12.5, 25, 62.5, 125, 250, 625, and 1000 nmol/L.

(5) The microplate was shaken at a given rate (400 rpm) at 22˚C for 3 hours. Then, a mixed solution of each well was filtered through a glass fiber filter (trade name: Mulutiscreen™-FC, manufactured by Millipore), to separate the Compound I-6 attached to amyloid from the free Compound I-6.

(6) The glass fiber filter used for filtration of the mixed solution was washed with a 0.1 % bovine serum albumin-containing phosphate buffer (0.5 mL x 5), and then radioactivity of the glass fiber filter was measured with an autowell gamma system (manufactured by Aloka, Type: ARC-301B).

(7) A relation between the amount of the Compound I-6 binding to amyloid and the amount of added amyloid was evaluated from the measurement results of (6). Unspecific binding was determined using a sample to which the Compound 1-2 (non-RI labeled compound) was added to become 100 nM (at a final concentration) above in (4)

(Example I-7).

(8) Using [$^{125}$I]-IMPY synthesized in the above Reference Example I-6, the same procedure as the above (2) to (6) were carried out to obtain control data (Comparative Example I-1).

A relation between the concentration of amyloid in the sample solution and the radioactive count on the glass fiber filter measured above in (6) is shown in Fig. 1-11. The radioactivity on the glass fiber was increased proportionally to the concentration (addition amount) of amyloid (Example I-7). On the conditions of the present experiment, amyloid and the compound attached to amyloid are retained in the glass fiber. Therefore, the radioactive count on the glass fiber is a value reflecting the amount of the compound attached to amyloid, and the slope of the graph which plotted the radioactive count relative to the concentration of amyloid is a value which can be an index representing the intensity of binding to amyloid. Since the value of the radioactive count of Compound I-6 on the glass fiber was increased with increase of the concentration of amyloid, it has been suggested that Compound I-6 is a compound having a property of binding to amyloid. The slope of the line for Compound I-6 was greater than the slope for [$^{125}$I]-IMPY in the same plot, and thus it has been suggested that the affinity of Compound 1-6 with amyloid is stronger than [$^{125}$I]-IMPY which is known to exhibit a strong affinity with amyloid.
The above-mentioned results have implied that Compound 1-6 is highly capable of binding to amyloid.

Examples I-8 to I-12, Comparative Examples I-2 to I-6: Measurement of affinity with amyloid

Affinity of the present compounds with amyloid was examined by the following *in vitro* binding tests.
(1) A$\beta_{1-40}$ (Peptide Institute, INC.) was dissolved in phosphate buffer (pH 7.4.) and shaken at 37˚C for 62-72 hours, to obtain a 1 mg/mL suspension of aggregated Aβ (hereinafter referred to as amyloid suspension in this Example).

(2) According to the method described in a literature (Naiki, H., et al., Laboratory investigation 74, p.374-333 (1996)), the amyloid suspension was subjected to qualitative experiment based on fluorescence spectrophotometric method using Thioflavin T (manufactured by Fluka) to confirm that the aggregated Aβ obtained in (1) was amyloid (measurement conditions: excitation wavelength of 446 nm, and emission wavelength of 490 nm).

(3) According to the method described in a literature (Wang, Y., et al., J. Labeled Compounds Radiopharmaceut. 44, S239 (2001)), [$^{125}$I]2-(3'-iodo-4'-aminophenyl)benzothiazole (hereinafter referred to as [$^{125}$I]3'-I-BTA-0) was prepared from a labeling precursor 2-(4'-aminophenyl)benzothiazole, and dissolved in ethanol. As Congo Red, Thioflavin T and 6-methyl-2-[4'-(N,N-dimethylamino)phenyl]ibenzothiazole (hereinafter referred to as 6-Me-BTA-2), commercially available reagents were weighed and used as they were.

(4) 2-(3'-Iodo-4'-aminophenyl)benzothiazole (hereinafter referred to as 3'-I-BTA-0) and IMPY were synthesized according to the methods described in a literature (Mang. Y., et al., J. Labelled Compounds Radiopharmaceut. 44, S239 (2001)) and a literature (Zhuang, Z. P., et al., J. Med. Chem. 46, 237 (2003)) respectively.

(5) Samples in which [$^{125}$I]3'-I-BTA-0, each compound for evaluation and amyloid were dissolved in a 0.1 % bovine serum albumin-containing phosphate buffer (pH 7.4) at final concentrations shown in Table 1-2 were prepared. The resulting samples were filled in each well (about 0.3 mL in volume) of a 96-well microplate.

Table 1-2: Finial concentration of each compound in sample solutions

| Experiment | Compound for evaluation | Concentration of compound for evaluation | [$^{125}$I]3'-I-BTA-0 concentration | Amyloid |
|---|---|---|---|---|
| Comparative Example I-2- | 3'-I-BTA-0 | Each concentration of 0, 0.001, 0.01, 0.1, 1, 10, 100, and 1000 nmol/L | 400 pmol/L | 1 μmol/L |
| Comparative Example I-3 | Congo Red | | | |
| Comparative Example I-4 | Thioflavin T | | | |
| Comparative Example I-5 | 6-Me-BTA-2 | | | |
| Comparative Example I-6 | IMPY | | | |
| Example I-8 | Compound I-1 | | | |
| Example I-9 | Compound I-2 | | | |
| Example 1-10 | Compound I-3 | | | |
| Example I-11 | Compound 1-4 | | | |
| Example I-12 | Compound I-5 | | | |

(6) The microplate filled with the sample solutions was shaken at a given rate (400 rpm) at 22°C for 3 hours. Then, each sample solution was filtered through a glass fiber filter (trade name: Mulutiscreen™-FC, manufactured by

Millipore), to separate the [$^{125}$I]3'-I-BTA-0 attached to amyloid from the free [$^{125}$I]3'-I-BTA-0.

(7) The glass fiber filter used for the filtration of each sample solution was washed with a 0.1 % bovine serum albumin-containing phosphate buffer (pH 7.4) (0.5 mL x 5), and radioactivity of the glass fiber filter was measured with an autowell gamma system (manufactured by Aloka, Type: ARC-301B), The radioactivity was used as the radioactivity level of each sample solution for calculating an inhibition ratio (hereinafter A denotes the radioactivity level in a sample with zero (0) concentration of each compound for evaluation, and B denotes the radioactivity level in a sample with 0.001 nmol/L or higher concentration of each compound for evaluation).

(8) Separately, a solution of containing 15 μmol/L of 6-HG-BTA-2, 400 pmol/L of [$^{125}$I]3'-I-BTA-0 and 1 μmol/L of $A\beta_{1-40}$ were prepared and subjected to the same procedures as described above in (6) and (7) to measure a radioactivity level. The Measured radioactivity level was defined as the background radioactivity level, and used in the calculation of the inhibition ratio (hereinafter referred to as BG).

(9) Using the radioactivity levels measured above in (7) and (8), the inhibition ratio was determined by the following formula (1-1).

$$\frac{B-BG}{A-BG}\times 100 \qquad (\%)\cdots(1-1)$$

A graph in which values converted by probit transformation from the obtained inhibition ratios were plotted relative to logarithms of concentrations of compounds for evaluation was prepared to obtain an approximate straight line by the least square method. Using the line, the concentration of each compound for evaluation was determined, at which the radioactivity level is half of the level of the sample free from each compound for evaluation, and was defined as a 50 % inhibition concentration of each compound (hereinafter referred to as $IC_{50\%}$ value). Using the value as an indicator, affinity of each compound for evaluation with amyloid (aggregated $A\beta_{1-40}$) was evaluated.

$IC_{50\%}$ value of each compound for evaluation is shown in Table 1-3. Compounds I-1 to I-5 all showed $IC_{50\%}$ values of less than 100 and had higher affinity with amyloid (aggregated $A\beta_{1-40}$) than Congo Red and Thioflavin T. The results show that Compounds I-1 to I-5 have good affinity with amyloid (aggregated $A\beta_{1-40}$). In particular, Compounds I-1 to I-4 had higher affinity with amyloid (aggregated $A\beta_{1-40)}$ than 3'-I-BTA-0 and 6-Me-BTA-2 and had the affinity comparable to IMPY.

Table 1-3: $IC_{50\%}$ values of the present compounds

| Experiment | Compound for evaluation | $IC_{50\%}$ values (nmol/L) |
|---|---|---|
| Comparative Example I-2 | 3'-I-BTA-0 | 10.1 |
| Comparative Example I-3 | Congo Red | >1000 |
| Comparative Example I-4 | Thioflavin T | >10000 |
| Comparative Example I-5 | -6-Me-BTA-2 | 25.4 |

(continued)

| Experiment | Compound for evaluation | $IC_{50\%}$ values (nmol/L) |
|---|---|---|
| Comparative Example I-6 | IMPY | 0.8 |
| Example I-8 | Compound I-1 | 1.4 |
| Example I-9 | Compound I-2 | 0.9 |
| Example I-10 | Compound I-3 | 1.8 |
| Example I-11 | Compound I-4 | 0.9 |
| Example I-12 | Compound I-5 | 55.4 |

Example I-13 to I-14, Comparative Example I-7: Measurement of partition coefficient based on the octanol extraction method

Partition coefficients based on the octanol extraction method (hereinafter referred two as $\log P_{octanol}$) were measured, which are generally known as an indicator of permeability of compounds through the blood-brain barrier (hereinafter referred to as BBB).

To 2 mL of octanol, 10 μL of a solution containing Compound I-7 (Example I-13) and Compound I-8 (Example I-14) and 2 mL of 10 mmol/L phosphate buffer (pH 7.4) were added, and stirred for 30 seconds. After the mixture was centrifuged with a low-speed centrifuge (2000 rpm x 60 min.), the octanol layer and the water layer were sampled each in an amount of 1 mL, and subjected to measurement of radioactivity count with an autowell gamma system (manufactured by Aloka, Type: ARC-301B). Using the obtained radioactivity count, $\log P_{octanol}$ was calculated in accordance with the equation (1-2).

$$\log P_{octanol} = \log_{10}\left(\frac{\text{Radioactivity count of octanol layer}}{\text{Radioactivity count of water layer}}\right) \cdots (1-2)$$

The results are shown in Table 1-4. Both Compounds I-7 and I-8 showed $\log P_{octanol}$ values between 1 and 3. It is known that compounds permeable to BBB show a $\log P_{octanol}$ value between 1 and 3 (Douglas D. Dischino et al., J. Nucl. Med., (1983), 24, p.1030-1038). Thus, it is implied that both compounds have a BBB permeability comparable to IMPY.

Table 1-4: $\log P_{octanol}$ value of the present compound

| Experiment | Compound | $\log P_{octanol}$ value |
|---|---|---|
| Comparative Example I-7 | [$^{123}$I]-IMPY | 2.1 |
| Example I-13 | Compound I-7 | 2.1 |
| Example I-14 | Compound I-8 | 2.0 |

Example I-15 to I-19, Comparative Example I-8: Measurements of partition coefficient based on HPLC

The partition coefficient by HPLC (hereinafter referred to as $\log P_{HPLC}$) was measured by the following method. It is known that the $\log P_{HPLC}$ shows the same numerical value at a pH of 7.2 to 7.4 as the $\log P_{octanol}$ value which is generally known as an indicator of permeability of compounds to BBB (Franco Lombardo et al., J. Med. Chem., (2000), 43, p.2922-2927).

First, compounds for evaluation shown in Table 1-5 were dissolved at a concentration of 1 mg/mL in methanol containing 10% dimethylsulfoxide to prepare sample solutions. One μL of the sample solution was subjected to HPLC

analysis under the following conditions determine the elution time ($t_0$) of the solvent and the elution time ($t_R$) of each compound.

Table 1-5 Compounds for evaluation in experiments

| Experiment | Compound for evaluation |
|---|---|
| Comparative Example I-8 | IMPY |
| Example I-15 | Compound I-1 |
| Example I-16 | Compound I-2 |
| Example I-17 | Compound I-3 |
| Example I-18 | Compound I-4 |
| Example I-19 | Compound I-5 |

HPLC conditions:

Column: Prodigy QDS (3) (product name; manufactured by phenomenex; size: 4,6 x 250 mm)
Mobile phase: a mixed solution of 50 mM triethylamine phosphate (pH 7.2)/acetonitrile = 40/60
Flow rate: 0.7 mL/min.
Detector: ultraviolet visible absorptiometer (detection wavelength: 282 nm)

Using the obtained to and $t_R$, the retention factor (hereinafter referred to as $K'_{HPLC}$ value) of each compound for evaluation was determined according to the following calculation formula (1-3).

$$K'_{HPLC} = (t_R - t_0)/t_0 \quad \cdots (1-3)$$

Separately, 10 μL each of a solution of 2-(4'-hydroxyphenyl)-6-[$^{125}$I]iodoimidazo[1,2-a]pyridine (37 MBq/mL in radioactivity concentration) synthesized above in Reference 1-8 and a solution of Compound 1-6 (37 MBq/mL in radioactivity concentration) was added to 2 mL of octanol prepared separately, and 2 mL of 10 mmol/L phosphate buffer (pH 7.4) was further added to the respective solutions. After the individual solutions were stirred for 30 seconds, the solutions were centrifuged at 2,000 rpm for 60 minutes. 1 mL each of the octanol phase, and the water phase was fractionated, and the radioactivity was measured by an autowell gamma system (manufactured by Aloka Co., Lid.: Type ARC-301B). Based on the obtained radioactivity, $logP_{octanol}$ values were calculated according to the above equation (1-2).

Further, a solution of Compound I-2 and 2-(4'-hydroxyphenyl)-6-iodoimidazo[1,2-a]pyridine prepared above in Reference I-9 were each subjected to HPLC analysis in the same manner as described above to determine $K'_{HPLC}$ values in respective compounds.

A graph was prepared, in which the $logP_{octanol}$ values of Compound I-6 and 2-(4'-hydroxyphenyl)-6-[$^{125}$I]iodoimidazo[1,2-a]pyridine are respectively plotted relative to the $log_{10}K'_{HPLC}$ values of Compound I-2 and 2-(4'-hydroxyphenyl)-6-iodoimidazo[1,2-a]pyridine, so that the slope and the intercept on the axis Y of the straight line were determined. Using these values, the following formula (1-4) was determined, provided that the $logP_{octanol}$ value is equal to the $logP_{HPLC}$ value at a pH of 7.2 to 7.4.

$$logP_{HPLC} = 0.96\left(log_{10}K'_{HPLC}\right) + 1.59 \quad \cdots (1-4)$$

Using $K'_{HPLC}$ obtained for each compound for evaluation, the $logP_{HPLC}$ value of each compound for evaluation was determined according to the above calculation formula (1-4).

The results are shown in Table 1-6. As shown in the Table, the $logP_{HPLC}$ values of Compounds I-1 through I-5 were all between 1 and 3. As mentioned above, it is known that compounds permeable to BBB have a $logP_{octanol}$ value between 1 and 3 (Douglas D. Dischino et al., J. Nucl. Med., (1983), 24, p. 1030-1038). Further, as mentioned above, it is known that the $logP_{HPLC}$ shows the same value at a pH of 7.2 to 7.4 as the $logP_{octanol}$ (Franco Lombardo et al., J. Med. Chem., (2000), 43, p.2922-2927). The above-mentioned results imply that Compounds I-1 to I-5 have a BBB-permeable property.

Table 1-6: $logP_{HPLC}$ value of the present compound

| Experiment | Compound | $logP_{HPLC}$ value |
|---|---|---|
| Comparative Example I-8 | IMPY | 2.1 |
| Example I-15 | Compound I-1 | 2.0 |
| Example I-16 | Compound I-2 | 2.1 |
| Example I-17 | Compound I-3 | 1.9 |
| Example I-18 | Compound I-4 | 1.9 |
| Example I-19 | Compound I-5 | 1.8 |

Example I-20 to I-21, Comparative Example I-9: Measurement of transferability into brain and clearance

Using Compound 1-7 (Example I-20) and Compound I-8 (Example I-21), a time course change of radioactive accumulation in brain of male Wistar rats (7-week old) was measured.

0.05 mL (20-30 MBq/mL in radioactive concentration) of a solution of Compound I-7 (Example I-20) in a 10 mg/mL ascorbic acid-containing physiological saline solution, a solution of Compound I-8 (Example I-21) in a 10 mg/mL ascorbic acid-containing physiological saline solution and a solution of [$^{123}$I]-IMPY (Comparative Example I-9) prepared above in Reference Example I-7 in a 10 mg/mL ascorbic acid-containing physiological saline solution were each injected under thiopental anesthesia into the tail vein of the rats. The rats were sacrifices by bleeding from abdominal artery, and brains were removed and subjected to measurement of radioactivity (hereinafter referred to as A in this Example) with an autowell gamma system (Type: ARC-301B manufactured by Aloka Co., Ltd.) and further subjected to measurement of mass of brains 2, 5, 30 and 60 minutes after injection. Also, radioactivity (hereinafter referred to as B in this Example) of 0.05 mL of a 1000-fold diluted solution of the injected solution was measured in the same manner as above. Using these measurement results, radioactive distribution per unit weight of brain (%ID/g) at the respective time points was calculated in accordance with the following formula (1-5).

Three animals were used for Experiment I-20 and Comparative Experiment I-9, and two animals were used for Experiment I-21 at the respective time points.

$$\%ID/g = \frac{A}{B \times 1000 \times brain\ weight} \times 100 \quad \cdots (1\text{-}5)$$

The results are shown in Table 1-7. As shown in Table 1-7, Compounds I-7 and I-8 showed an accumulation higher than [$^{123}$I]-IMPY at the time point of two minutes after the injection, and then showed a tendency to rapidly clear away in 60 minutes. These results suggest that Compounds I-7 and I-8 possess excellent transferability to brain and rapid clearance from brain comparable to [$^{123}$I]-IMPY.

Table 1-7: Radioactive distribution in brain of the present compound after intravenous injection (rats)

| Compound | | Radioactive distribution per unit weight (%ID/g) | | | |
|---|---|---|---|---|---|
| | | After 2 min. | After 5 min. | After 30 min. | After 60 min. |
| Example I-20 | Compound I-7 | 1.10 | 0.75 | 0.12 | 0.05 |
| Example I-21 | Compound I-8 | 1.20 | 0.75 | 0.18 | 0.12 |
| Comparative Example I-9 | $^{123}$I-IMPY | 1.02 | 0.99 | 0.20 | 0.08 |

Example I-22: Confirmation of imaging of amyloid in brain

The following experiment was carried out in order to examine whether amyloid in brain can be imaged by the compound of the present invention.

(1) $A\beta_{1-40}$ (manufactured by Peptide Institute, INC.) was dissolved in phosphate buffer (pH 7.4) and shaken at 37˚C for 72 hours, to obtain 1 mg/mL of a suspension of aggregated Aβ (hereinafter referred to as amyloid suspension in this Example).

(2) 25 μL (corresponding to 25 μg) of the amyloid suspension was injected into an amygdaloid nucleus on one side of a male Wistar rat (7-week old). As go control, 25 μL of a phosphate buffered physiological saline solution (pH 7.4) was injected into an amygdaloid nucleus on the other side of the rat. The rats were examined 1 day after the injection of the amyloid suspension and the phosphate buffered physiological saline solution (pH 7.4).

(3) Compound I-7 was dissolved in a 10 mg/mL ascorbic acid-containing physiological saline solution to obtain a sample solution (32 MBq/mL in radioactivity concentration). This solution was injected into the rat through the tail vein (dosage: 0.5 mL, dosed radioactivity: 16 MBq equivalent).

(4) Brain was removed 60 minutes after the injection to prepare a brain slice of 10 μm in thickness with a microtome (type: CM3050S, manufactured by LEICA). The brain slice was exposed to an imaging plate for 20 hours, and then image analysis was carried out by use of a Bio-imaging Analyzer (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelehgth: 470 nm). Thus, it was confirmed that amyloid was deposited on the slice (Fig. 1-12b).

Fig. 1-12 shows images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was injected. From the result of Thioflavin T staining in the site where radioactivity is accumulated, it was confirmed that amyloid was present in the accumulation site. On the other hand, no significant accumulation of radioactivity was observed in the amygdaloid nucleus or the side to which the physiological saline solution was injected, compared with the other sites.

These results suggest that Compound I-7 possesses a property of accumulating on intracerebral amyloid and a capability of imaging intracerebral amyloid.

Example I-23 to I-26: Reverse mutation test

In order to examine mutagenicity of Compound I-1, I-2, I-4 and I-5, reverse mutation test using *Salmonella typhimurium* TA98 and TA100 (hereinafter referred to as Ames test) was conducted.
The test was conducted without addition of S9mix and with addition of S9mix. Dimethylsulfoxide was used as a negative control. A positive control was 2-(2-furyl)-3-(5-nitro-2-furyl)acrylamide in case S9mix was not added, and 2-aminoanthracene in case S9mix was added.
The amount of each sample to be added to the test plate was 7 dosages (geometric ratio 4) with the maximum dose being 1250 μg/plate for Compounds I-1 and I-5, and 7 dosages (geometric ratio 3) with the maximum dose being 5000 μg/plate for Compounds I-2 and I-4. After a sample to be examined and a strain (TA98 or TA100), or a sample to be examined, S9mix and the strain were mixed together, the mixture was multilayered using soft agar on a medium of a test plate, and then incubated at 37˚C for 48 hours. Judgment was made by counting the number of reverse mutation colonies on the plate after the incubation, and when the number of reverse mutation colonies was not less than two times the number in negative control and showed concentration-dependent increase, mutagenicity was determined to be positive.
The results are shown in Table 1-8. The numbers of reverse mutation colonies of the respective strains in the group treated with Compounds I-1, I-2, I-4 and I-5 were less than two times the number in the group treated with the negative control, regardless of addition of S9mix and the addition amount of a sample to be examined. From the a forementioned results, it is judged that Compounds I-1, I-2, I-4 and I-5 are negative in the Ames test and have no mutagenicity.
Table 1-8: Results of Ames test

| | Compound | Mutagenicity | | | |
|---|---|---|---|---|---|
| | | Without addition of S9mix | | With addition of S9mix | |
| | | TA98 | TA100 | TA98 | TA100 |
| Example I-23 | Compound I-1 | Negative | Negative | Negative | Negative |
| Example I-24 | Compound I-2 | Negative | Negative | Negative | Negative |
| Example I-25 | Compound I-4 | Negative | Negative | Negative | Negative |
| Example I-26 | Compound I-5 | Negative | Negative | Negative | Negative |

Example I-27: Synthesis of 6-tributylstannyl-2-[4'-(2"-fluoroethoxy)pheny]imidazo[1,2-alpyridine

88 mg (corresponding to 0.260 mmol) of 6-bromo-2-[4'-(2"-fluoroethoxy)phenyl]imidazo[1,2-a]pyridine obtained in Reference Example 1-3 was dissolved in 10.0 mL of dioxane, and 2.0 mL of triethylamine was added thereto. Then, 0.20 mL (corresponding to 0.39 mmol) of bis(tributyltin) and 20.1 mg (a catalytic amount) of tetrakis-triphenylphosphine palladium were added whereto. After the reaction mixture was stirred at 90˚C for 9 hours, the solvent was distilled off under reduced pressure. The residue was purified by flash silica gel column chromatography (elution solvent: hexane/ethyl acetate = 4/1) to obtain 71.6 mg (corresponding to 0.131 mmol) of 6-tributylstannyl-2-[4'-(2"-fluoroethoxy)phenyl] imidazo[1,2-a]pyridine (Fig. 1-13, Step 1.)
The NMR measurement results of the obtained 6-tributylstannyl-2-[4'-(2"-fluoroethoxy)phenyl]imidazo[1,2-a] pyridine (internal standard: tetramethylsilane) are shown below.
NMR apparatus employed: JNM-ECP-500 (manufactured by Japan Electron Optics laboratory Co., Ltd. (JEOL)) $^1$H-NMR (solvent: chloroform-dl; resonance frequency: 500 MHz): δ 7.97 (s, 1H), 7.90 (d, J = 8.7 Hz, 2H), 7.58 (d, J = 8.7 Hz, 1H), 7.14 (d, J = 8.7 Hz, 1H), 6.99 (d, J = 8.7 Hz, 2H), 4.77, (dt, J = 47.2, 4.1 Hz, 2H), 3.99 (dt, J = 28.0, 4.1 Hz, 2H), 1.59-1.53 (m, 6H), 1.39-1.32 (m, 6H), 1.13-1.10 (m, 6H), 0.92 (t, J = 7.3 Hz, 9H).

$^{13}$C-NMR (solvent: chloroform-dl, resonance frequency: 500 MHz): δ 158.3, 145.6, 144.9, 131.2, 130.0, 127.4, 121.9, 116.9, 114.9, 106.4, 82.6, 81.3, 67.2, 29.0, 27.3, 13.6, 9.8.

Example I-28: Synthesis of 2-[4'-(2"-fluoroethoxy)phenyl-6-[$^{123}$I]iodoimidazo[1,2-a]pyridine

To 35 μL of a solution of 6-tributylstannyl-2-[4'-(2"-fluoroethpxy)phenyl]imidazo[1,2-a]pyridine in methanol (concentration: 1 mg/mL), 100 μL of 1 mol/L hydrochloric acid, [$^{123}$I] sodium iodide of 614 MBq (100 μL in volume), 10 μL of 1 mol/L sodium iodide solution and 20 μL of 10% (w/v) hydrogen peroxide were added. After the mixed solution was heated at 50°C for 10 minutes, the solution was subjected to HPLC under the same conditions as in Example I-2, to obtain 2-[4'-(2"-fluoroethoxy)phenyl]-6-[$^{123}$I]iodoimidazo[1,2-a]pyridine fraction.

10 ml of water was added two the fraction. The resulting solution was passed through a reversed phase column (trade name: Sep-Pak (registered trademark) Light C8 Cartridges manufactured by Waters: the packed amount of the packing agent: 145 mg) so that the column adsorbs and collects 2-[4'-(2"-fluoroethoxy)phenyl]-6-[$^{123}$I] iodoimidazo[1,2-alpyridine. The column was rinsed with 1 mL of water, and then 1 mL of ethanol was passed therethrough to elute 2-(4'-(2"-fluoroethoxy)phenyl]-6-[$^{123}$I]iodoimidazo[1,2-a]pyridine. The amount of radioactivity of the obtained compound was 64 MBq at the end of synthesis. Further, the TLC analysis was conducted under the following conditions, and as a result, the radiochemical purity of the compound was 97.0%.

TLC analysis conditions:

TLC plate: Silica Gel 60 $F_{254}$ (trade name; manufactured by Merck & Co., Inc.)
Mobile phase: Chloroform/methanol/trlethylamine = 100/1/2 Detector: Rita Star (trade name; manufactured by raytest)

Example I-29, Comparative Example I-10: Measurement of partition coefficient based on the octanol extraction method

A diethyl ether solution of Compound I-9 prepared in Example I-28 (Example 1-29) and a diethyl ether solution of [$^{123}$I]-IMPY (Comparative Example 1-10) were each diluted with a 10 mg/mL ascorbic acrid-containing physiological saline solution, and adjusted radioactive concentration to be 20-30 MBq/mL. To 2 mL of octanol, 10 μL each of the prepared sample solutions was added, and 2 mL of a 10 mmol/L phosphate buffer (pH 7.4) was further added, followed by stirring for 30 seconds. After the mixture was centrifuged with a low-speed centrifuge (2000 rpm x 60 min.), the octanol layer and the water layer Mere sampled each in an amount of 1 mL, and subjected to measurement of radioactivity count with an autowell gamma system (Type: ARC-301B, manufactured by Aloka). Using the obtained radioactivity count, $\log P_{octanol}$ was calculated in accordance with the equation (1-6).

$$\log P_{octanol} = \log_{10}\left(\frac{\text{Radioactivity count of octanol layer}}{\text{Radioactivity count of water layer}}\right) \cdots (1-6)$$

The results are shown in Table 1-9. Compound I-9 also showed a $\log P_{octanol}$ value between 1 and 3. It is known that compounds permeable to BBB show a $\log P_{octanol}$ value between 1 and 3 (Douglas D. Dischino et al., J. Nucl. Med., (1983), 24, p.1030-1038). Thus, it is implied that Compound I-9 has a BBB-permeability comparable to IMPY.

Table I-9: $\log P_{octanol}$ value of the present compound

| Experiment | Compound | $\log P_{octanol}$ value |
|---|---|---|
| Comparative Example I-10 | [$^{123}$I]-IMPY | 2.1 |
| Example I-29 | Compound I-9 | 2.1 |

Example I-30, Comparative Example I-11: Measurement of transferability into brain and clearance (2)

Using Compound I-9, a time course change of radioactive accumulation in brain of male Wistar rats (7-week old) was measured.

A solution of Compound I-9 (Example I-30) and a solution of [$^{123}$I]-IMPY (Comparative Example I-11) prepared

above in Reference Example each in a 10 mg/mL ascorbic acid-containing physiological saline solution (20-31 MBq/mL in radioactive concentration) were prepared. 0.05 mL each of these solutions was injected under thiopental anesthesia into the tail vein of respective Wistar rats (7-week old). The rats were sacrificed by bleeding from abdominal artery, and brains were removed and subjected to measurement of mass of brains and further subjected to measurement of radioactivity (hereinafter referred to as A in this Example) with a single channel analyzer (detector type: SP-20 manufactured by OHYO KOKEN KOGYO Co., Ltd.) 2, 5, 30 and 60 minutes after the injection. Also, radioactivity (hereinafter referred to as B in this Example) of the rest of the whole body was measured in the same manner as above. Using these measurement results, radioactive distribution per unit weight of brain (%ID/g) at the respective time points were calculated in accordance with the following formula (1-7).
Three animals were used for experiment at the respective time points.

$$\%ID/g = \frac{\mathrm{A}}{\mathrm{B} \times brain\ weight} \times 100 \quad \cdots (1\text{-}7)$$

The results are shown in Table 1-10. As shown in Table 1-10, Compound 1-9 showed a significant radioactive accumulation like [$^{123}$I]-IMPY at the time point of two minutes after the injection, and then showed a tendency to rapidly clear away in 60 minutes. These results suggest that Compound I-9 possesses excellent transferability to brain and rapid clearance from brain like [$^{123}$I]-IMPY.

Table 1-10: Radioactive distribution in brain of the present compound after intravenous injection (rates)

| Compound | | Radioactive distribution per unit weight (%ID/g) | | | |
|---|---|---|---|---|---|
| | | After 2 min. | After 5 min. | After 30 min. | After 60 min. |
| Example I-30 | Compound I-9 | 0.72 | 0.49 | 0.07 | 0.02 |
| Comparative Example I-11 | $^{123}$I-IMPY | 1.19 | 0.97 | 0.23 | 0.09 |

Example I-31: Confirmation of imaging of amyloid in brain

(1) A$\beta_{1-42}$ (Wako) was dissolved in phosphate buffer (pH 7.4) and shaken at 37°C for 72 hours, to obtain a 1 mg/mL suspension of aggregated Aβ (hereinafter referred to as amyloid suspension in this Example).

(2) 2.5 μL (corresponding to 25 μg) of the amyloid suspension was injected into an amygdaloid nucleus on one side of a male wistar rat (7-week old). As a control, 2.5 μL of a phosphate buffered physiological saline solution (pH 7.4) was injected into an amygdaloid nucleus on the other side of the rat. The rats were examined 1 day after the injection of the amyloid suspension and the phosphate buffered physiological saline solution (pH 7.4).

(3) Compound I-9 was dissolved in a 10 mg/mL ascorbic acid-containing physiological saline solution to obtain a sample solution (21 MBq/mL in radioactivity concentration in a sample solution, Example I-31). This solution Was injected under thiopental anesthesia into the rat through the tail vein (dosage: 0.5 mL, dosed radioactivity: 11-15 MBq equivalent).

(4) Brain was removed 60 minutes after the injection to prepare a brain slice of 10 μm in thickness with a microtome (type: CM3050S, manufactured by LEICA). The brain slice was exposed to an imaging plate for 20 hours, and then image analysis was carried out by use of a Bio-imaging Analyzer (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 1-14).

Fig. 1-14 shows images by autoradiogram and Thioflavin T staining of the brain slice of the rat to which amyloid was injected intracerebrally. As shown in this figure, a marked accumulation of radioactivity in the specimen to which Compound 1-9 was injected was also observed in the amygdaloid nucleus on the side to which the amyloid suspension was injected. On the other hand no significant accumulation of radioactivity was observed in the amygdaloid nucleus on the side to which the physiological saline solution was injected, compared with the other sites. On the autoradiogram, little accumulation of radioactivity was observed at sites other than the sites to which amyloid was injected. From the result of Thioflavin T staining, it was confirmed that amyloid was present in the site where radioactivity is accumulated (Fig. 1-14). These results imply that Compound 1-9 possesses a property of accumulating on intracerebral amyloid and a capability of imaging intracerebral amyloid.

Example I-32: Chromosome Aberration Test

In order to examine whether Compound I-4 can induce chromosome aberration, the chromosome aberration test was conducted using Chinese Hamster fibroblast cell line (CHL/IU cell) in a culture system with or without addition of S9 by short-term treatment process and in a 24-hour culture system by continuous treatment process. The addition amount of a sample to be tested was set to 1.2, 0.6, 0.3, and 0.15 mg/mL for all the respective culture systems.

When appearance frequency of cells with chromosome aberration apparently increased as compared two negative control group and dose dependency was observed, or when the appearance frequency apparently increased at single dose and reproducibility was observed, positive determination was given, and otherwise negative determination was given.

As the results of the test, the appearance frequency of cells having structural aberration or numerical aberration (diploid) in all the culture systems treated with Compound I-4 was comparable to that of the negative control group. On the other hand, the positive control group for the respective culture systems showed a marked increase of the appearance frequency of cells having structural aberration. From the above results, the capability of inducing chromosome aberration of Compound I-4 was judged to be negative under the test conditions.

Example I-33: Micronucleus Test

In order to study mutagenicity (*in vino*) of Compound I-4, induction of micronucleated polychromatic erythrocyte (hereinafter referred to as MNPCE) was examined using bone narrow cells of Crlj:CD1(ICR) male

0 mg/kg (negative control group), 250, 500, 1000 and 2000 mg/kg (test sample group) were set as doses for the test. Mice wore sacrificed 24 and 48 hours after single oral administration, and bone marrow smear was prepared and observed.. Further, a single dose of MMC at 2 mg/kg was abdominally administrated in positive control group, mice were sacrificed 24 hours after the administration, and then bone marrow smear was prepared and observed.

When appearance frequency of MNPCE in each administration group showed a dose-dependent increase or a statistically significant increase as compared to negative control group, positive determination was given, and otherwise negative determination was given. As statistical analysis, significance tests were conducted by Wilcoxon rank sum test for the appearance frequency of MNPCE and the ratio of polychromatic erythrocyte (hereinafter referred to as PCE) to the total red blood cell (hereinafter referred to as RBC) in each administration group concerning the test sample and positive control groups relative to the negative control group and concerning these groups relative to each other Where the significance level was set to less than 5% and less than 1%, respectively.

From the results of the test, no statistically significant difference was observed on the appearance frequency of MNPCE and the ratio of PCE to RBC for the test sample group as compared to the negative control group. On the other hand, a significant increase was observed on the appearance frequency of MNPCE for the positive control group as compared to the negative control group. Based on these results, mutagenicity (*in vino*) of the test sample was judged to be negative because no induction of micronucleus in mouse bone marrow cells was observed under the above test

conditions using Compound I-4.

(Example II-1 to Example II-2) Confirmation of binding to amyloid

In order to study a binding mechanism of a compound of the present invention, an experiment for inhibition of binding to Thioflavin T was conducted using amyloid derived from amylin as a precursor compound. Amylin is an amyloid accumulating in pancreas of type II diabetes.

Method

(1) Amylin (human) (manufactured by Wako) was dissolved in phosphate buffer (pH 7.4) and shaken at 37˚C for 72 hours, to obtain a 1 mg/mL suspension of aggregated amylin (hereinafter referred to as amyloid suspension in this Example).

(2) According to the method described in a literature (Naiki, H., et al., Laboratory Investigation 74, p.374-383 (1996)), the amyloid suspension was subjected to qualitative experiment based on fluorescence spectrophotometric method using Thioflavin T (manufactured by Fluka) to confirm that the aggregated amylin obtained in (1) was amyloid (measurement conditions excitation wavelength of 446 nm, and fluorescence wavelength of 490 nm).

(3) The amyloid suspension was dissolved if 50mM phosphate buffer (pH 7.4) at a concentration of amylin of 15 μM. Also, Thioflavin T was dissolved at a concentration of 15 μM in 0 mM glycin-NaOH buffer (pH 8.5).

(4) Samples in which each compound for evaluation and amyloid were dissolved in a 50 mM phosphate buffer (pH 7.4) at final concentrations shown in Table 2-1 were prepared. 50 μL of each amyloid solution and Thioflavin T solution prepared in (3), and 50 μL of a sample solution were placed in each well (about 0.3 mL in volume) of a 96-well microplate.

Table 2-1: Final concentrations of each compound in sample solutions

| Experiment | Compound for evaluation | Concentration of compound for evaluation | Thioflavin T concentration | Amyloid |
|---|---|---|---|---|
| Example II-1 | Compound I-1 | Each concentration of 0, 1.5, 15, 30 μmol/L | 5 μmol/L | 2.6 μmol/L |
| Example II-2 | Compound I-2 | | | |

(5) A sample in which 100 μL of 50 mM phosphate buffer (pH 7.4) and 50 μL of 50 mM glycin-NaoH buffer (pH 8.5) were mixed was made as a blank, and subjected to the same procedure as in (4) and used for a calculation of inhibition ratio.

(6) The microplate filled with the sample solutions was left to stand still for 30 hours. Then, fluorescence strength

of each sample solution was measured (measurement conditions; excitation wavelength of 4.46nm, emission wavelength of 490 nm) with microplate reader (type: SPECTRA MAX GEMINI XS, manufactured by Molecular Devices) (hereinafter, A denotes the fluorescence strength in a sample with zero (0) concentration of each compound for evaluation, and B denotes the fluorescence strength in a sample with 1.5 μmol/L or higher concentration of each compound for evaluation).

(7) Using the fluorescence strength measured above in (6), the following formula (2-1):

$$\text{Inhibition Ratio} = \frac{B-BG}{A-BG}\times 100 \quad (\%) \quad \cdots (2\text{-}1)$$

was used to determine the inhibition ratio.

Inhibition ratio of each compound, for evaluation is shown in Table 2-2. Compounds I-1 and I-2 both prevented a binding of Thioflavin T at any concentration. The results showed that Compounds I-1 and I-2 competitively inhibit a binding of Thioflavin T. It is generally known that Thioflavin T binds with recognition of β-sheet structure of amyloid. Thus, it has been suggested that Compounds I-1 and I-2 has a same mechanism of binding: to amyloid as Thioflavin T, namely, binds thereto with recognition of β-sheet structure.

Table 2-2: Inhibition ratio (%) of binding of amyloid (amylin) to Thioflavin T by the present compounds

| Experiment | Compound for evaluation | Inhibition ratio (%) | | |
|---|---|---|---|---|
| | | Concentration of compound for evaluation 1.5 μmol/L | Concentration of compound for evaluation 15 μmol/L | Concentration of compound for evaluation 30 μmol/L |
| Example II-1 | Compound I-1 | 14.5 | 37.0 | 43.7 |
| Example II-2 | Compound I-2 | 28.9 | 26.6 | 28.2 |

(Example III) Measurement of radioactive distribution ratio in each organ

In order to confirm that a compound of the present invention can be distributed in a target organ and has good clearance to the outside of the body. Compound I-9 was used to measure a time-course change of radioactive accumulation to each organ in SD rat (8 week).

To 100 μL of a solution of 6-tributylstannyl-2-[4'-(2"-fluoroethoxy)phenyl]imidazo[1,2-a]pyridine in acetonitrile (concentration: 1 mg/mL), 100 μL of 1 mol/L sulfuric acid, 10 μL of 1 mmol/L sodium iodide, 60 μL of [$^{123}$I]sodium iodide of 981 MBq, and 10 μL of 30% (w/v) hydrogen peroxide were added. After the mixed solution was left to stand at 40°C for 10 minutes, the solution was subjected to HPLC under the following conditions, to obtain [$^{123}$I]-2-[4'-(2"-fluoroethoxy) phenyl-6-iodoimidazo[1,3]pyridine as a fraction.

HPLC conditions:

Column: YMC-Pack Pro C8 (trade name; manufactured by YMC;
size: 4.6 x 150 mm)
Mobile phase: 10 mM formic acid ((pH 3. 0) /acetonitrile = 80/20 to 90/0 (0 minute to 30 minutes)
Flow rate: 1.0 mL/min.
Detector: Ultraviolet visible absorptiometer (Detection wavelength: 254 nm) and radioactivity counter (manufactured by raytest: type STEFFI)

10 ml of water was added to the fraction. The resulting solution was passed through a Sep-Pak C18 column (trade name: Sep-Pak (registered trademark) Light C18 Cartridges manufactured by Waters: the packed amount of the packing agent: 130 mg) so that the column adsorbs and [$^{123}$I] -2- [4'-(2"-fluoroethoxy)phenyl]-6-iodoimidazo[1,2-a]pyridine. The column was rinsed with 1 mL of water, and then 1 mL of diethyl ether was passed therethrough to elute [$^{123}$]-2-[4'-(2"-fluoroethoxy)phenyl]-6-iodoimidazo[1,2-a]pyridine (Compound 1-9). The amount of radioactivity of the obtained compound was 473 MBq at the end of the synthesis. Further, the TLC analysis was conducted under the following conditions, and as a result, the radiochemical purity of the compound was 98%.

TLC analysis conditions:

TLC plate: Silica Gel 60 $F_{254}$ (trade name; manufactured by Merck & Co., Inc.)
Mobile phase: ethyl acetate/methanol/diethylamine = 100/4/1
Detector: Rita Star (trade name; manufactured by raytest)

A solution of Compound I-9 in diethylether was diluted with a 10 mg/mL ascorbic acid-containing physiological saline solution and adjusted to 8-12 MBq/mL in radioactive concentration. 0.2 mL each of the prepared sample solution was administered under non-thiopental anesthesia into the tail vein of the above rat. The rats were sacrificed by bleeding from abdominal artery, and each organ shown in Table 3 was removed 5, 30, 60 and 180 minutes after the administration. Each removed organ was subjected to measurement of mass and radioactivity with the same method as in Example I-30. Also, radioactivity of the whole body of the rat after removal of the organs (hereinafter referred to as rest of the whole body) was measured. Using those measurement results, radioactive distribution per unit weight of each organ (%ID/g) at the respective time points was calculated in accordance with the following formula (3).
Three animals were used for the experiment at the respective time points.

$$\%ID/g = \frac{R^T}{(R^S + R^R) \times M^T} \times 100 \cdots (6)$$

$R^T$: Radioactivity of target organ (cpm)
$R^S$: Sum of radioactivity of all the organs (cpm)
$R^R$: Radioactivity of the rest of the whole body (cpm)
$M^T$: Mass of target organ (g)

The results are shown in Table 3. As shown in Table 3, Compound I-9 was distributed to each organ at the time point of 5 minutes after administration, and then most of the radioactivity was distributed to small intestine and large intestine. In addition, radioactive distribution thereof was transferred from small intestine to large intestine. Thus, Compound I-9 was found to be biliary-excreted rapidly after the administration and have a good clearance to the outside of

the body.
Also, observing brain, heart, lung, pancreas and bone in which amyloid is considered to accumulate, obvious radioactive accumulation was found in all these organs at the time point of 5 minutes after the administration, and thus distribution of Compound I-9 was confirmed. Moreover, the ratio of the time point of 5 minutes after the administration to the time point of 180 minutes after the administration ((%ID/g at the time point, of 5 minutes after the administration) / (%ID/g at the time point of 180 minutes after the administration)) showed high values such as 94 for brain, 18 for heart, 9 for lung, 9 for pancreas and 6 for bone. Thus, it has been shown that rapid radioactive distribution and rapid clearance are performed in organs in which amyloid is considered to accumulate.
From the above, it has been shown that a radioactive distribution at an early stage after administration and a rapid clearance to the outside of the body, which are required for an amyloid detecting reagent in a biological tissue, have been attained.
Table 3

| Tissue/ organ | 5 min. after administration | | 30 min. after administration | | 60 min. after administration | | 180 min. after administration | |
|---|---|---|---|---|---|---|---|---|
| | Average | Standard deviation | Average | Standard deviation | Average | Standard deviation | Average | Standard deviation |
| Blood | 0.370 | 0.010 | 0.142 | 0.013 | 0.108 | 0.005 | 0.074 | 0.012 |
| Brain | 0.701 | 0.039 | 0.067 | 0.010 | 0.026 | 0.004 | 0.007 | 0.001 |
| Heart | 0.551 | 0.036 | 0.111 | 0.007 | 0.059 | 0.004 | 0.030 | 0.006 |
| Lung | 0.647 | 0.067 | 0.192 | 0.032 | 0.130 | 0.013 | 0.074 | 0.010 |
| Liver | 2.399 | 0.130 | 0.337 | 0.019 | 0.178 | 0.022 | 0.067 | 0.008 |
| Spleen | 0.442 | 0.029 | 0.092 | 0.005 | 0.069 | 0.007 | 0.037 | 0.007 |
| Pancreas | 0.650 | 0.027 | 0.236 | 0.022 | 0.115 | 0.012 | 0.073 | 0.053 |
| Stomach | 0.287 | 0.077 | 0.619 | 0.268 | 0.672 | 0.042 | 0.812 | 0.196 |
| Small intestine | 1.259 | 0.306 | 6.598 | 0.212 | 8.235 | 0.654 | 2.657 | 1.024 |
| Large intestine | 0.106 | 0.022 | 0.070 | 0.007 | 0.074 | 0.018 | 5.241 | 0.684 |
| Kidney | 1.114 | 0.112 | 0.560 | 0.073 | 0.302 | 0.034 | 0.108 | 0.026 |
| Adrenal gland | 3.914 | 0.521 | 0.457 | 0.090 | 0.260 | 0.014 | 0.017 | 0.030 |
| Bone | 0.187 | 0.019 | 0.067 | 0.009 | 0.045 | 0.007 | 0.032 | 0.006 |
| Marrow | 0.815 | 0.211 | 0.075 | 0.066 | 0.000 | 0,000 | 0.000 | 0.000 |
| Muscle | 0.239 | 0.008 | 0.052 | 0.004 | 0.031 | 0.000 | 0.016 | 0.003 |

## INDUSTRIAL APPLICABILITY

The reagent for detecting amyloid in biological tissues according to the present invention can be utilized as diagnosing agents for amyloid protein *in vitro* and *in vivo* in amyloidosis such as systemic amyloidosis.

## BRIFF DESCRIPTION OF THE DRAWINGS

Fig. 1-1 is a scheme of synthesis of 6-tributylstannyl-2-[4'- (3"-fluoropropoxy)phenyl]imidazo[1,2-a]pyridine.
Fig. 1-2 is a scheme of synthesis of 6-bromo-2-[4'-(3"-p-toluenesulfonyloxypropoxy)phenyl]imidazo[1,2-a]pyridine.
Fig. 1-3 is a scheme of synthesis of 6-bromo-2-[4'-(2"-p-toluenesulfonyloxyethoxy)phenyl]imidazo[1,2-a]pyridine.
Fig. 1-4 is a scheme of synthesis of 6-bromo-2-[4'-(3-fluoropropoxy)phenyl]imidazol[1,2-a]pyridine.

Fig. 1-5 is a scheme of synthesis of 2-[4'-(3"-fluoropropoxy)phenyl]-6-iodoimidazo[1,2-a]pyridine.
Fig. 1-6 is a scheme of synthesis of 6-bromo-2-[4'-(2"-fluoroethoxy)pheyl]imidazo[1,2-a]pyridine.
Fig. 1-7 is a scheme of synthesis of 2-[4'-(2"-fluoroethoxy)phenyl]-6-iodoimidazo[1,2-a]pyridine.
Fig. 1-8 is a scheme of synthesis of 2-[4'-(3"-fluoropropoxy)phenyl]-6-iodoimidazo[1,2-a]pyrimidine.
Fig. 1-9 is a scheme of synthesis of [$^{125}$I]-2-(4'-hydroxyphenyl)-6-iodoimidazo[1,2-a]pyridine.
Fig. 1-10 is a scheme of synthesis of 2-(4'-hydroxyphenyl)-6-iodoimidazo[1,2-a]pyridine.
Fig. 1-11 is a relationship between amyloid concentration and radioactive concentration in sample solutions.
Fig. 1-12(a) is an autoradiogram of the brain slice after the injection of Compound I-7, and Fig, 1-12(b) is a fluorescent microscopic image of the Thioflavin T stained sample (a magnification of the site to which the amyloid suspension was injected).
Fig. 1-13 is a scheme of synthesis of 6-tributylstannyl-2-[4'-(2"-fluoroethoxy)phenyl]imidazo[1,2-a]pyridine.
Fig. 1-14(a) is an autoradiogram of the brain slice the injection of Compound I-9, and Fig. 1-14(b) is a fluorescent microscopic image of the Thioflavin T stained sample (a magnification of the site to which the amyloid suspension was injected).

## Claims

**1.** A reagent for detecting amyloid deposited in a biological tissue, which comprises a compound represented by the following formula (1), or a salt thereof:

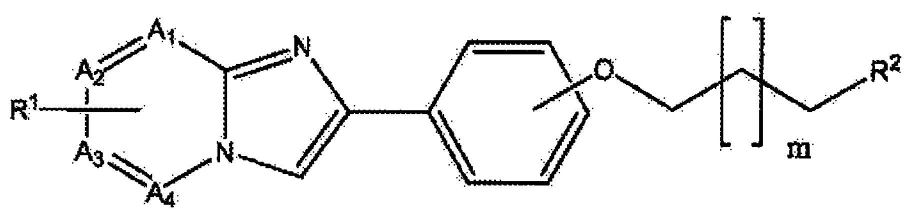

(1)

wherein $A_1$, $A_2$, $A_3$ and $A_4$ independently represents a carbon or nitrogen,
$R^1$ is a halogen substituent,
$R^2$ is a halogen substituent, and
m is an integer of 0 to 2,
provided that at least one of $R^1$ and $R^2$ is a radioactive halogen substituent, at least one of $A_1$, $A_2$, $A_3$ and $A_4$ represents a carbon, and $R^1$ binds to a carbon represented by $A_1$, $A_2$, $A_3$ or $A_4$.

**2.** A reagent according to claim 1, wherein at least three of $A_1$, $A_2$, $A_3$ and $A_4$ represent carbons.

**3.** A reagent according to claim 2, wherein all of $A_1$, $A_2$, $A_3$ and $A_4$ represent carbons.

**4.** A reagent according to any one of claims 1 to 3, wherein $R^1$ is selected from the group consisting of $^{18}$F, $^{79}$Br, $^{76}$Br, $^{123}$I, $^{124}$I, $^{125}$I and $^{31}$I.

**5.** A reagent according to any one of claims 1 to 4, wherein $R^3$ is selected from the group consisting of $^{18}$F, $^{75}$Br $^{76}$Br, $^{123}$I, $^{124}$I, $^{125}$I and $^{131}$I.

**6.** A reagent according to any one of claims 1 to 5, wherein the biological tissue is brain, heart, lung, pancreas, bone, or joint.

**7.** A process for production of a radioactive halogen-labeled organic compound, which comprises a step of preparing a reaction solution containing, together with a radioactive halogen ion, a compound represented by the following formula (2) or a salt thereof:

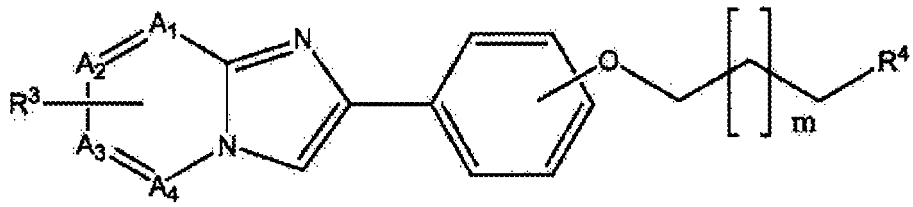

(2)

wherein $A_1$, $A_2$, $A_3$ and $A_4$ independently represents a carbon or nitrogen,
$R^3$ is a group selected from the group consisting of a non-radioactive halogen substituent nitro substituent, trialkylammonium substituent having alkyl chains with 1 to 4 carbon atoms, trialkylstannyl substituent having alkyl chains with 1 to 4 carbon atoms and triphenylstannyl group,
$R^9$ is a group selected from the group consisting of a non-radioactive halogen substituent, methanesulfonyloxy substituent, trifluoromethanesulfonyloxy substituent and aromatic sulfonyloxy substituent, and
m is an integer of 0 to 2,
provided that at least one of $A_1$, $A_2$, $A_3$ and $A_4$ represents a carbon, and $R^3$ binds to a carbon represented by $A_1$, $A_2$, $A_3$ or $A_4$, and
a step of giving a reaction condition to the above reaction solution to synthesize a compound represented by the following formula (1) or a salt thereof:

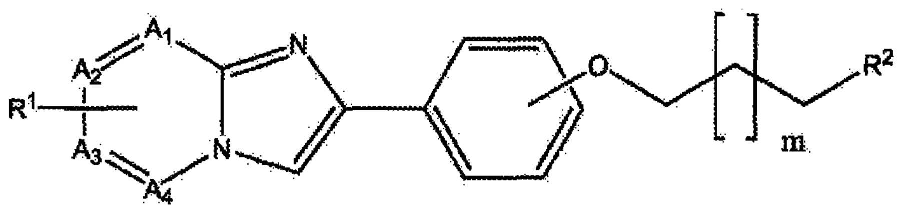

(1)

wherein $A_1$, $A_2$, $A_3$ and $A_4$ independently represents a carbon or nitrogen,
$R^1$ is a halogen substituent,
$R^2$ is a halogen substituent, and
m is an integer of 0 to 2,
provided that at least one of $R^1$ and $R^2$ is a radioactive
halogen substituent, at least one of $A_1$, $A_2$, $A_3$ and $A_4$ represents a carbon and $R^1$ binds to a carbon represented by $A_1$, $A_2$, $A_3$ or $A_1$.

**8.** A process for production of a radioactive halogen-labeled organic compound, according to claim 7, wherein at least three of $A_1$, $A_2$, $A_3$ and $A_4$ represent carbons.

**9.** A process for production of a radioactive halogen-labeled organic compound, according to claim 8, wherein all of $A_1$, $A_2$, $A_3$ and $A_4$ represent carbons.

**10.** A process for production of a radioactive halogen-labeled organic compound, according to any one of claims 7 to 9, wherein $R^3$ is selected from the group consisting of a non-radioactive iodine, trialkylstannyl substituent having alkyl chains with 1 to 4 carbon atoms and triphenylstannyl groups
$R^2$ and $R^4$ are non-radioactive halogen substituents, the radioactive halogen ion is selected from the group consisting of 123I ion, $^{134}I$ ion, $^{125}I$ ion and $^{131}I$ ion, and $R^1$ is selected from the group consisting of $^{123}I$, $^{124}I$ $^{125}I$ and $^{131}I$.

**11.** A process for production of a radioactive halogen-labeled organic compound, according to any one of claims 7 to 9, wherein $R^3$ is selected from the group consisting of a nitro substituent or trialkylammonium substituent having alkyl chains with 1 to 4 carbon atoms, $R^2$ and $R^4$ are non-radioactive halogen substituent, the radioactive halogen ion is $^{18}F$ ion, and $R^1$ iS $^{18}F$.

**12.** A process for production of a radioactive halogen-labeled organic compound, according to any one of claims 7 to 9, wherein $R^3$ is a non-radioactive bromine, $R^2$ and $R^4$ are non-radioactive halogen substituents,

the radioactive halogen ion is $^{75}Br$ ion or $^{76}Br$ ion, any $R^1$ is $^{75}Br$ or $^{76}Br$.

**13.** A process four production of a radioactive halogen-labeled organic compound, according to any one of claims 7 to 9, wherein $R^1$ and $R^3$ are non-radioactive halogen substituents,
$R^4$ is selected from the group consisting of methanesulfonyloxy substituent, trifluoromethanesulfonyloxy substituent and aromatic sulfonyloxy substituent,
the radioactive halogen ion is $^{18}F$ ion, and $R^2$ is $^{18}F$.

**14.** A process for production of a radioactive halogen-labeled organic compound, according two any one of claims 7 to 9, wherein $R^1$ and $R^3$ are non-radioactive halogen substituents,
$R^4$ is a non-radioactive iodine,
the radioactive halogen substituent is selected from the group consisting of $^{123}I$ ion, $^{124}I$ ion, $^{125}I$ ion and $^{132}I$ ion, and $R^2$ is selected from the group consisting of $^{125}I$, $^{124}I$, $^{125}I$ and $^{131}I$.

**15.** A process for production of a radioactive halogen-labeled organic compound according to any one of claims 7 to 9, wherein $R^1$ a and $R^3$ are non-radioactive halogen substituent,
$R^4$ is non-radioactive bromine,
the radioactive halogen ion is $^{75}Br$ ion or $^{76}Br$ ion, and $R^2$ is $^{75}Br$ or $^{76}Br$.

**16.** A precursor compound for synthesizing a radioactive halogen-labeled organic compound, which is represented by the following formula (2) or a salt thereof:

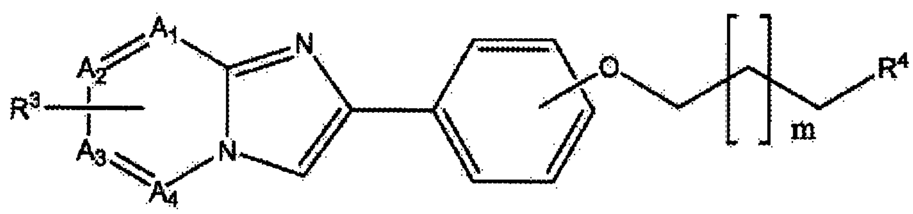

(2)

wherein $A_1$, $A_2$, $A_3$ and $A_4$ independently represents a carbon or nitrogen,
$R^3$ is a group selected from the group consisting of a non-radioactive halogen substituent, nitro substituent, trialkylammonium substituent having alkyl chains with 1 to 4 carbon atoms, trialkylstannyl substituent having alkyl chains with 1 to 4 carbon atoms and triphenylstannyl group,
$R^4$ is a group selected from the group consisting of a non-radioactive halogen substituent, methanesulfonyloxy substituent, trifluoromethanesulfonyloxy substituent and aromatic sulfonyloxy substituent, and
m is an integer of 0 to 2,
provided that at least, one of $A_1$, $A_2$, $A_3$ and $A_4$ represents a carbon, and $R^3$ binds to a carbon represented by $A_1$, $A_2$, $A_3$ or $A_4$.

**17.** A precursor compound for synthesizing a radioactive halogen-labeled organic compound or a salt thereof, according to claim 16, wherein at least three of $A_1$, $A_2$, $A_3$ and $A_4$ represent carbons.

**18.** A precursor compound for synthesizing a radioactive halogen-labeled organic compound or a salt thereof, according to claim 17, wherein all of $A_1$, $A_2$, $A_3$ and $A_4$ represent carbons.

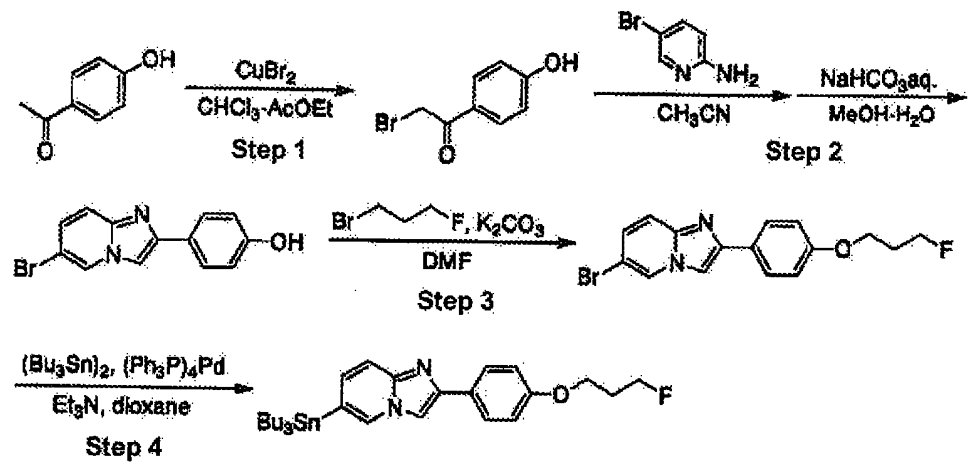


Figure 1-1

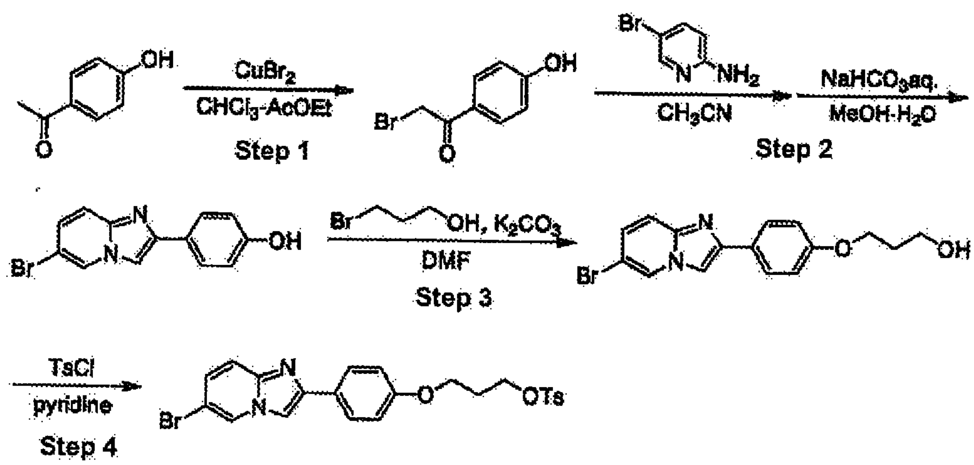


Figure 1-2

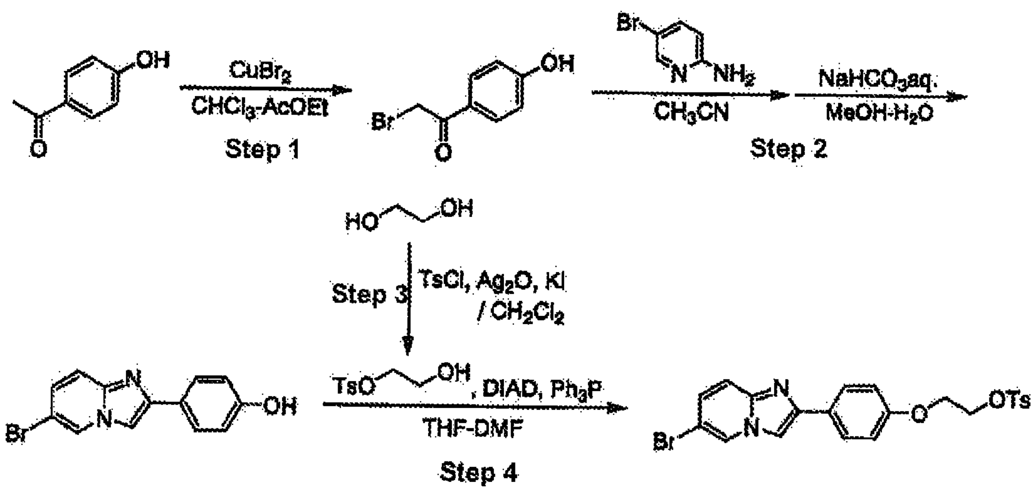


Figure 1-3

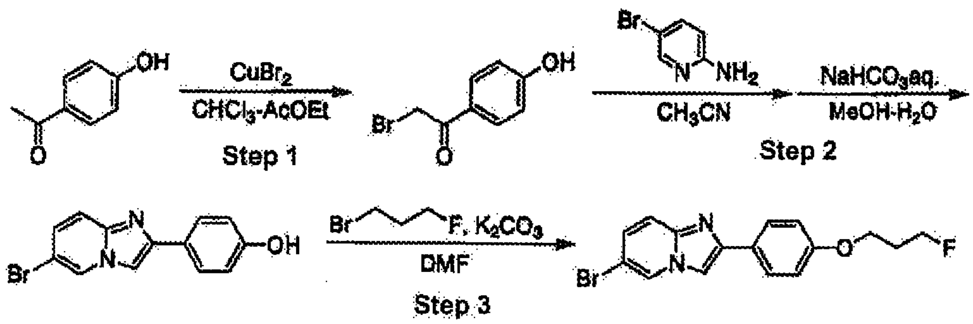


Figure 1-4

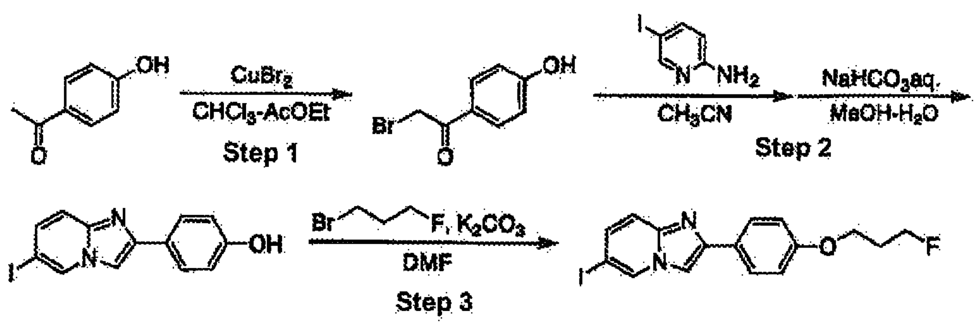


Figure 1-5

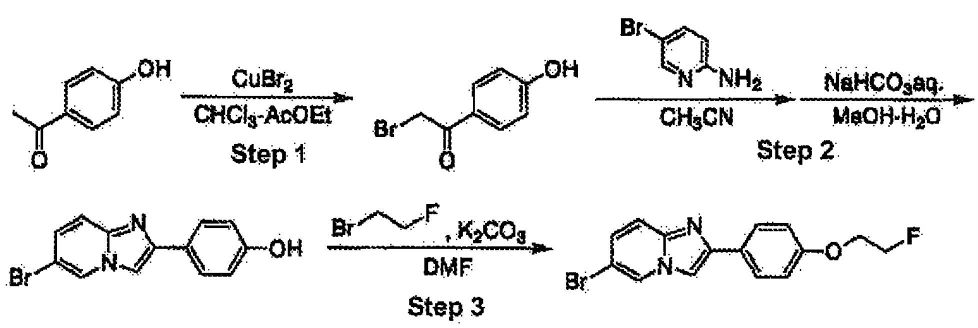


Figure 1-6

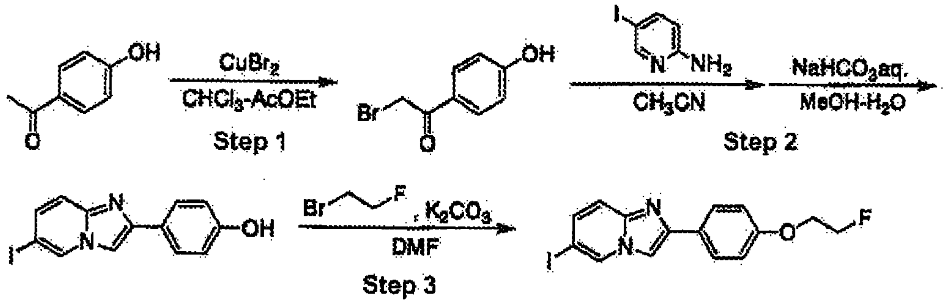


Figure 1-7

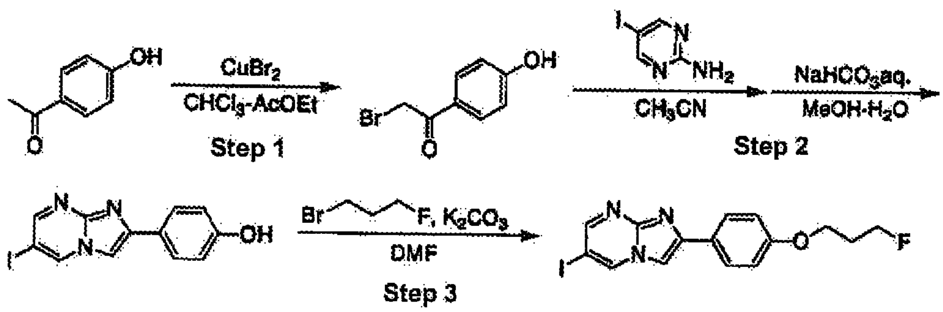


Figure 1-8

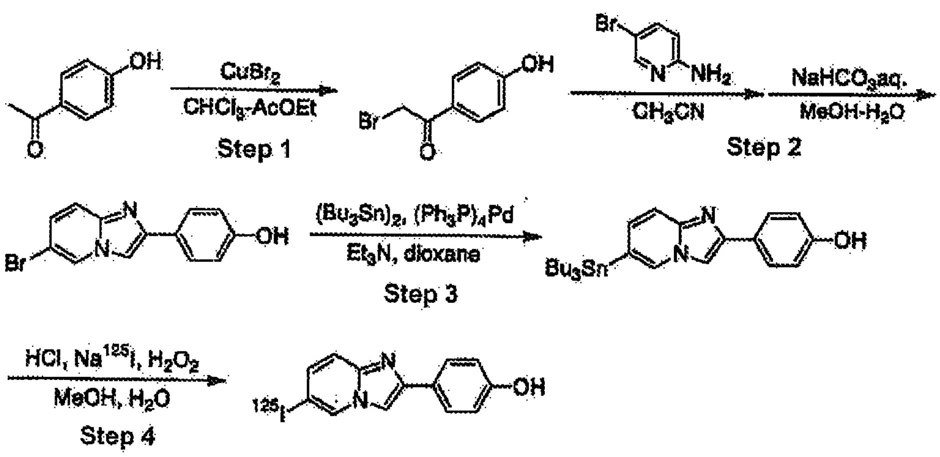


Figure 1-9

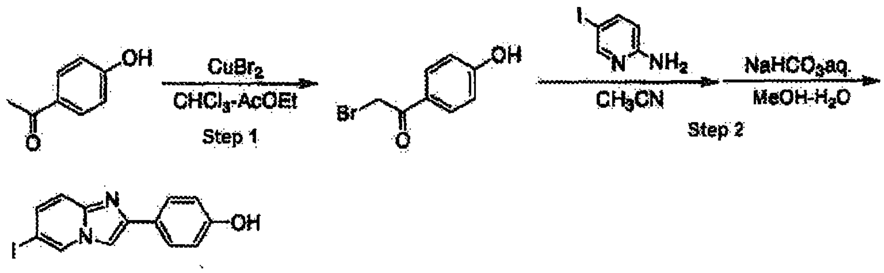


Figure 1-10

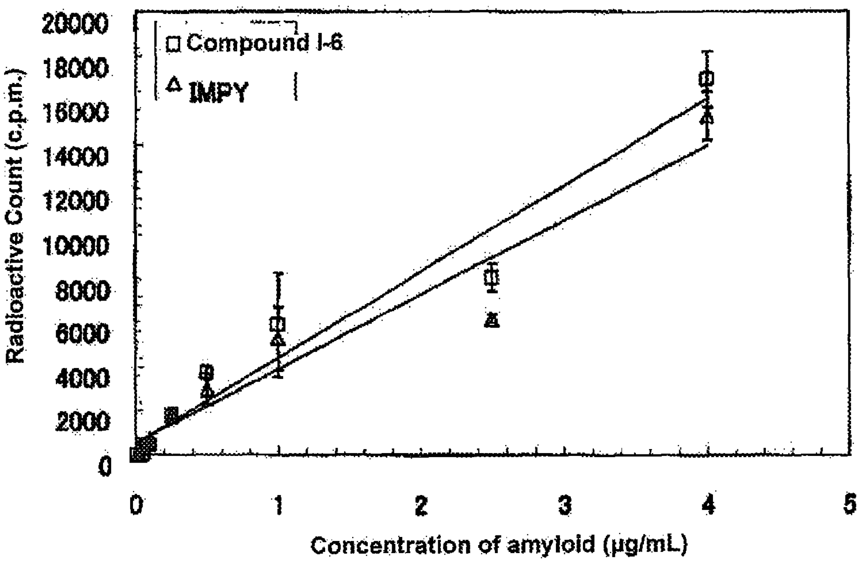


Figure 1-11

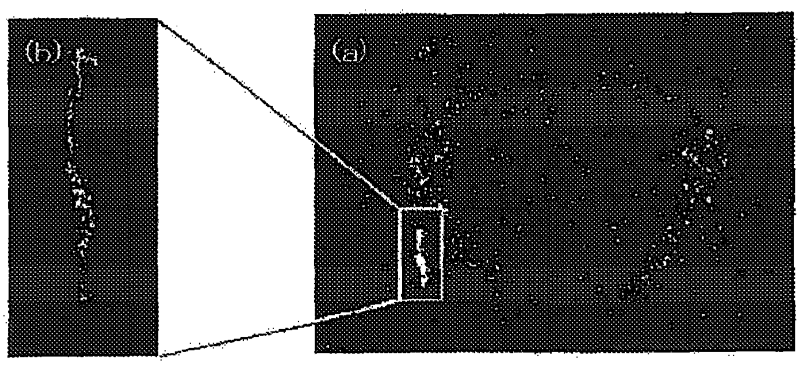


Figure 1-12

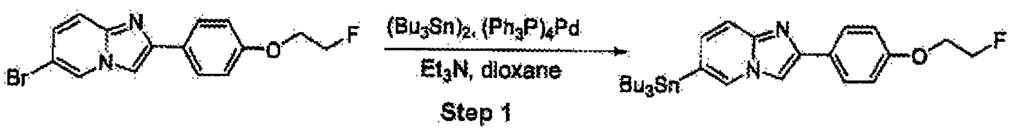


Figure 1-13

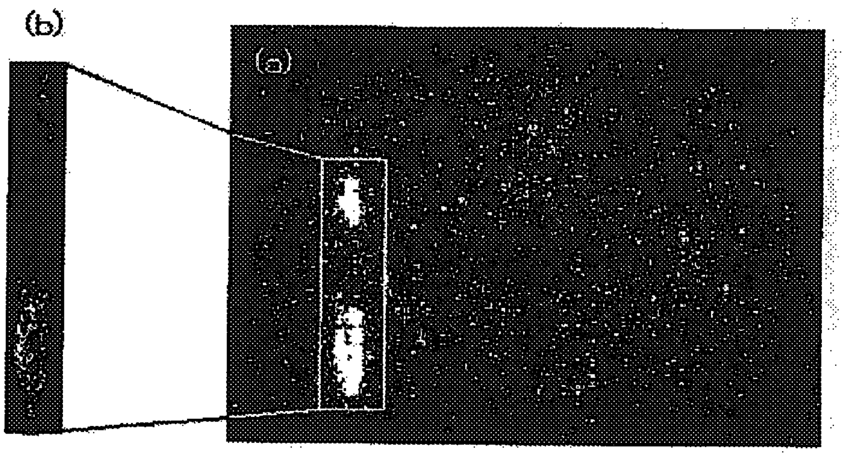


Figure 1-14

**INTERNATIONAL SEARCH REPORT**

International application No.
PCT/JP2008/069502

A. CLASSIFICATION OF SUBJECT MATTER
*A61K51/00*(2006.01)i, *C07D471/04*(2006.01)i

According to International Patent Classification (IPC) or to both national classification and IPC

B. FIELDS SEARCHED

Minimum documentation searched (classification system followed by classification symbols)
A61K51/00, C07D471/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>A | JP 63-91391 A (Ortho Pharmaceutical Corp.), 22 April, 1988 (22.04.88), Claims; page 7, lower column; example 3<br>& US 4727145 A & US 4791117 A<br>& US 4833149 A & US 4871745 A<br>& EP 261912 A2 | 16-18<br>1-15 |
| P,X | WO 2007/135890 A1 (Nihon Medi-Physics Co., Ltd.), 29 November, 2007 (29.11.07), Full text<br>(Family: none) | 1-18 |

☒ Further documents are listed in the continuation of Box C.  ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

Date of the actual completion of the international search
21 November, 2008 (21.11.08)

Date of mailing of the international search report
02 December, 2008 (02.12.08)

Name and mailing address of the ISA/
Japanese Patent Office

Facsimile No.

Authorized officer

Telephone No.

Form PCT/ISA/210 (second sheet) (April 2007)

**INTERNATIONAL SEARCH REPORT**

International application No. PCT/JP2008/069502

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-512945 A (The Trustees of the University of Pennsylvania), 12 May, 2005 (12.05.05), & WO 2002/085903 A2 & EP 1381604 A2 & US 2004/0131545 A1 | 1-18 |
| A | WO 2001/074813 A2 (ORTHO MCNEIL PHARMACEUTICAL, INC.), 11 October, 2001 (11.10.01), & US 2001/0051632 A1 & EP 1268478 A | 1-18 |
| A | JP 2004-525192 A (F. Hoffmann-La Roche AG.), 19 August, 2004 (19.08.04), & US 2003/0212096 A1 & EP 1381363 A & WO 2002/092086 A1 | 1-18 |
| A | JP 2001-43978 A (Mitsui Chemicals, Inc.), 16 February, 2001 (16.02.01), (Family: none) | 1-18 |
| A | WO 2005/066177 A1 (SCHERING PLOUGH LTD.), 21 July, 2005 (21.07.05), & US 2005/0182059 A1 & EP 1699799 A & WO 2005/066177 A1 | 1-18 |
| A | JP 2005-526749 A (Novartis AG.), 08 September, 2005 (08.09.05), & US 2005/0169837 A1 & EP 1483262 A & WO 2003/074519 A1 | 1-18 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007002540 A
- WO 2007063946 A
- JP 2004506723 T
- JP 2005604055 T
- JP 2005512945 T
- JP 2002523383 T
- WO 2005016888 A
- WO 03106439 A

### Non-patent literature cited in the description


- **J. A. Hardy ; G. A. Higgins.** Alzheimer's Disease: The Amyloid Cascade Hypothesis. *Science,* 1992, vol. 256, 184-185
- **G. McKhahn et al.** Clinical diagnosis of Alzheimer's disease: Report of the NINCDS-ADRDA Work Group under the auspices of Department of Health and Human Services Task Force on Alzheimer's Disease. *Neurology,* 1984, vol. 34, 939-944
- **Z.-P. Zhuang et al.** Radioiodinated Styrylbenzenes and Thioflavins as Probes for Amyloid Aggregates. *J. Med. Chem.,* 2001, vol. 44, 1905-1514
- **Masahiro Ono et al.** 11C-labeled stilbene derivatives as Aβ-aggregate-specific PET imaging agents for Alzheimer's disease. *Nuclear Medicine and Biology,* 2003, vol. 30, 565-571
- **H. F. Kung et al.** Novel Stilbenes as Probes for amyloid plaques. *J. American Chemical Society,* 2001, vol. 123, 12740-12741
- **Zhi--Ping Zhuang et al.** IBOX (2-(4'-dimethylaminophenyl)-6-iodobensoxazole): a ligand for imaging amyloid plaques in the brain. *Nuclear Medicine and Biology,* 2001, vol. 28, 887-894
- **Furumoto Y et al.** [11C]BF-227: A New C-Labeied 2-Ethenylbenzoxazole Derivative for Amyloid-β Plaques Iniaging. *European Journal of Nuclear Medicine and Molecular Imaging,* 2005, vol. 32 (1), 759
- **Eric D. Agdeppa et al.** 2-Dialkylamimp-6-Acylamlononitrile Substituted Naphthalenes (DDNP Analogs): Novel Diagnostic and Therapeutic Tools in Alzheimer's Disease. *Molecular Imaging and Biology,* 2003, vol. 5, 404-417
- **Zhi-Ping Zhuang et al.** Structure-Activity Relationship of Imidazo[1,2-a]pyridines as Ligands for Detecting β-Amyloid Plaques in the Brain. *J. Med. Chem,* 2003, vol. 46, 237-243
- **W. E. Klunk et al.** Imaging brain amyloid in Alzheimer's disease with Pittsburgh Compound-B. *Ann. Neurol.,* 2004, vol. 55, 306-319
- **Nicolaas P. L. G. Verhoeff et al.** In-Vivo Imaging of Alzheimer Disease β-Amyloid with [11C]SB-13 PET. *American Journal of Geriatric Psychiatry,* 2004, vol. 12, 584-595
- **Hiroyuki Arai et al.** [11C]-BF-227 AND PET to Visualize Amyloid in Alzheimer's Disease Patients. *Alzheimer's & Dementia: The Journal of the Alzheimer's Association,* 2006, vol. 2 (1), 312
- **Christopher M. Clark et al.** Imaging Amyloid with I123 IMPY SPECT. *Alzheimer's & Dementia: The Journal of the Alzheimer's Association,* 2006, vol. 2 (1), 342
- **D. M. Skovronsky et al.** *Proc. Natl. Acad. Sci.,* 2000, vol. 97, 7609
- **Zhi-Ping Zhuang et al.** *Nuclear Medicine, and Biology,* 2001, vol. 28, 887-894
- **H. F. King.** *J. Am. Chem. Soc.,* 2001, vol. 123, 12740-13741
- **Masahiro Ono et al.** *Nuclear Medicine and Biology,* 2003, vol. 30, 565-571
- **King, L. Carroll ; Ostrum, G. Kenneth.** *Journal of Organic Chemistry,* 1964, vol. 29 (12), 3459-3461
- **Zhi-Ping Zhuang et al.** *J. Med. Chem,* 2003, vol. 46, 237-243
- **Zhi-Ping Zhuang et al.** *J. Med. Chem.,* 2003, vol. 46, 237-243
- **Naiki, H. et al.** *Laboratory Investigation,* 1996, vol. 74, 374-383
- **Naiki, H. et al.** *Laboratory investigation,* 1996, vol. 74, 374-333
- **Wang, Y. et al.** *J. Labeled Compounds Radiopharmaceut.,* 2001, vol. 44, 239
- **Mang. Y. et al.** *J. Labelled Compounds Radiopharmaceut.,* 2001, vol. 44, 239
- **Zhuang, Z. P. et al.** *J. Med. Chem.,* 2003, vol. 46, 237
- **Douglas D. Dischino et al.** *J. Nucl. Med.,* 1983, vol. 24, 1030-1038
- **Franco Lombardo et al.** *J. Med. Chem.,* 2000, vol. 43, 2922-2927